# EUROPEAN PATENT APPLICATION

(11) **EP 3 881 867 A1**
(43) Date of publication of application: **22.09.2021**
(21) Application number: 21163617.0
(22) Date of filing: 11.07.2011
(51) Int. Cl.: A61K 39/395, C07K 16/22

(54) **STABILIZED FORMULATIONS CONTAINING ANTI-NGF ANTIBODIES**

(30) Priority: 14.07.2010 US 36411210 P
(62) Divisional of application: 18188992.4
(71) Applicant: Regeneron Pharmaceuticals, Inc., Tarrytown, NY 10591 (US)
(72) Inventor: WALSH, Scott, Tarrytown, NY 10591 (US); POTOCKY, Terra, Dobbs Ferry, NW 10522 (US); DIX, Daniel, Lagrangeville, NY 12540 (US); SIVENDRAN, Renuka, Thousand Oaks, CA 91360 (US)
(74) Representative: J A Kemp LLP

(57) **Abstract**

The present invention provides pharmaceutical formulations comprising a human antibody that specifically binds to human nerve growth factor (hNGF). The formulations may contain, in addition to an anti-hNGF antibody, at least one non-ionic surfactant, at least one sugar, and acetate. The pharmaceutical formulations of the present invention exhibit a substantial degree of antibody stability after storage for several months.

## Description

### FIELD OF THE INVENTION

The present invention relates to the field of therapeutic antibody formulations. More specifically, the present invention relates to the field of pharmaceutical formulations comprising a human antibody that specifically binds to human nerve growth factor (NGF).

### BACKGROUND

Therapeutic macromolecules (e.g., antibodies) must be formulated in a manner that not only makes the molecules suitable for administration to patients, but also maintains their stability during storage. For example, therapeutic antibodies in liquid solution are prone to degradation, aggregation and/or undesired chemical modifications unless the solution is formulated properly. The stability of an antibody in liquid formulation depends not only on the kinds of excipients used in the formulation, but also on the amounts and proportions of the excipients relative to one another. Furthermore, other considerations aside from stability must be taken into account when preparing a liquid antibody formulation. Examples of such additional considerations include the viscosity of the solution and the concentration of antibody that can be accommodated by a given formulation. Thus, when formulating a therapeutic antibody, great care must be taken to arrive at a formulation that remains stable, contains an adequate concentration of antibody, and possesses a suitable viscosity as well as other properties which enable the formulation to be conveniently administered to patients.

Antibodies to the human nerve growth factor (hNGF) are one example of a therapeutically relevant macromolecule that requires proper formulation. Anti-NGF antibodies are clinically useful for the treatment and/or prevention of diseases such as osteoarthritis, sciatica, and other conditions such as inflammatory pain, neuropathic pain, and cancer pain.

Although anti-NGF antibodies are known, there remains a need in the art for novel pharmaceutical formulations comprising anti-hNGF antibodies, which are sufficiently stable and also suitable for administration to patients.

### BRIEF SUMMARY OF THE INVENTION

The present invention satisfies the aforementioned need by providing pharmaceutical formulations comprising a human antibody that specifically binds to human nerve growth factor (hNGF). The formulations of the invention may comprise excipients in addition to the anti-hNGF antibody. For example, in certain embodiments, the formulation may comprise (i) a human antibody that specifically binds to hNGF; (ii) a non-ionic surfactant; and (iii) at least one carbohydrate. The non-ionic surfactant may be selected from the group consisting of polysorbate 20, polysorbate 80, polyoxyethylene sorbitan monooleate, and polyethylene glycol. In one embodiment, the non-ionic surfactant is polysorbate 20. The carbohydrate can be a sugar such as, e.g., sucrose, glucose, mannitol, sorbitol, lactose or trehalose. In one embodiment, the sugar is sucrose.

In one embodiment, the pharmaceutical formulation comprises (i) about 0.1 to 100 mg/mL of a human antibody that specifically binds to hNGF; (ii) about 0.01 to 1.0 % of polysorbate 20; and (iii) about 1 to 20% sucrose.

In one embodiment, the pharmaceutical formulation comprises (i) about 0.2 to 75 mg/mL of a human antibody that specifically binds to hNGF; (ii) about 0.02 to 0.5 % of polysorbate 20; and (iii) about 5 to 10% sucrose.

In one embodiment, the pharmaceutical formulation comprises (i) about 0.6 to 60 mg/mL of a human antibody that specifically binds to hNGF; (ii) about 0.05% of polysorbate 20; and (iii) about 8% sucrose.

In certain embodiments, the pharmaceutical formulation further comprises about 1.0 mM to about 50 mM acetate.

In one embodiment, the pharmaceutical formulation comprises (i) about 1 to 100 mg/mL of a human antibody that specifically binds to hNGF; (ii) about 0.01 to 1.0 % of polysorbate 20; and (iii) about 1 to 20% sucrose. In certain embodiments, the pharmaceutical formulation further comprises about 1.0 mM to about 50 mM acetate.

In one embodiment, the pharmaceutical formulation comprises: (i) about 5 to 75 mg/mL of a human antibody that specifically binds to hNGF; (ii) about 0.02 to 0.5% polysorbate 20; (iii) about 5 to 10% sucrose; and (iv) about 5 to 20 mM acetate.

In one embodiment, the pharmaceutical formulation comprises: (i) about 6-60 mg/mL of a human antibody that specifically binds to hNGF; (ii) about 0.05% polysorbate 20; (iii) about 8% sucrose; and (iv) about 10 mM acetate.

In certain embodiments, the formulation may also contain at least one amino acid, e.g. histidine or arginine. In certain embodiments, the concentration of histidine or arginine may range from about 25 mM to about 100 mM.

According to certain embodiments of the present invention, the formulation is prepared in a buffer that is capable of maintaining a pH ranging from about pH 4.5 to about pH 5.6, for example, an acetate buffer.

In certain embodiments of the invention, the pharmaceutical formulation exhibits a viscosity of less than about 15 cPoise, or less than about 12 cPoise, or less than about 9 cPoise.

The antibody contained within the pharmaceutical formulations of the present invention can be any antibody, or a fusion protein or trap, which specifically binds to hNGF. Exemplary antibodies that may be contained within the formulations of the invention are antibodies comprising a heavy chain variable region (HCVR) and a light chain variable region (LCVR), wherein the HCVR comprises a heavy chain complementarity determining region (HCDR) 1 having the amino acid sequence of SEQ ID NO: 6, a HCDR2 having the amino acid sequence of SEQ ID NO: 8, and a HCDR3 having the amino acid sequence of SEQ ID NO: 10; and wherein the LCVR comprises a light chain complementarity determining region (LCDR) 1 having the amino acid sequence of SEQ ID NO: 14, a LCDR2 having the amino acid sequence of SEQ ID NO:16, and a LCDR3 having the amino acid sequence of SEQ ID NO:18.

In certain embodiments, the antibody contained within the formulations of the present invention is an antibody comprising a HCVR having the amino acid sequence of SEQ ID NO:20 and a LCVR having the amino acid sequence of SEQ ID NO:22.

In certain embodiments, the antibody contained within the formulations of the present invention is an antibody comprising a HCVR having the amino acid sequence of SEQ ID NO:4 and a LCVR having the amino acid sequence of SEQ ID NO:12.

In certain embodiments, the pharmaceutical formulations may be administered intravenously or subcutaneously to a patient in need thereof. Accordingly, in certain embodiments, the pharmaceutical formulation, as described herein, may be used in the preparation of a medicament for the treatment, prevention and/or amelioration of any disease or disorder associated with NGF activity or NGF activation. Exemplary, non-limiting diseases and disorders that can be treated and/or prevented by the administration of the pharmaceutical formulations of the present invention include, pain resulting from any condition associated with neurogenic, neuropathic or nociceptic pain. In certain embodiments of neuropathic pain, referred trigeminal neuralgia, post-herpetic neuralgia, phantom limb pain, fibromyalgia, reflex sympathetic dystrophy and neurogenic pain conditions are treated. In other embodiments, cancer pain, particularly, bone cancer pain, osteoarthritis or rheumatoid arthritis pain, lower back pain, post-operative incision pain, fracture pain, osteoporotic fracture pain, osteoporosis, gout joint pain, diabetic neuropathy, sciatica, pains associated with sickle cell crises, migraine, and other neuropathic and/or nociceptic pains are treated with the formulations described herein.

The antibody formulations of the present invention may be contained within any suitable container useful for storing pharmaceutical formulations. Examples of such suitable containers include, e.g., glass or plastic vials, syringes and cartridges. The container may be clear or opaque (e.g., amber colored). In certain embodiments, the vials or syringes are coated with silicone, such as silicone dioxide. In certain embodiments, the headspace in the vials are filled with an inert gas to displace any oxygen present that may have an adverse effect on stability of the antibody. Such inert gas may be selected from nitrogen or argon.

According to certain aspects of the present invention, the pharmaceutical formulations remain relatively stable following storage for several days, months or years at a given temperature. For example, in certain exemplary embodiments of the present invention, a high percentage of the antibody (e.g., 90%, 95%, 96% or more) is maintained in its native form following at least 3, 6, 9 or more months of storage. The percentage of native form of the antibody may be measured, e.g., by SE-HPLC, or by any other method known in the art. The storage temperature at which stability of the antibody is maintained can be, e.g., -80°C, -40°C, - 30°C, -20°C, 0°C, 5°C, 25°C, 37°C, 45°C, or higher.

Other embodiments of the present invention will become apparent from a review of the ensuing detailed description.

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 1%. For example, as used herein, the expression "about 100" includes 99 and 101 and all values in between (e.g., 99.1, 99.2, 99.3, 99.4, etc.).

Although any methods and materials similar or equivalent to those described herein can be used in the practice or testing of the present invention, the preferred methods and materials are now described.

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 1%. For example, as used herein, the expression "about 100" includes 99 and 101 and all values in between (e.g., 99.1, 99.2, 99.3, 99.4, etc.).

Although any methods and materials similar or equivalent to those described herein can be used in the practice or testing of the present invention, the preferred methods and materials are now described.

### PHARMACEUTICAL FORMULATIONS

As used herein, the expression "pharmaceutical formulation" means a combination of at least one active ingredient (e.g., a small molecule, macromolecule, compound, etc. which is capable of exerting a biological effect in a human or non-human animal), and at least one inactive ingredient which, when combined with the active ingredient and/or one or more additional inactive ingredients, is suitable for therapeutic administration to a human or non-human animal. The term "formulation," as used herein, means "pharmaceutical formulation" unless specifically indicated otherwise. The present invention provides pharmaceutical formulations comprising at least one therapeutic polypeptide. According to certain embodiments of the present invention, the therapeutic polypeptide is an antibody that binds specifically to human nerve growth factor (hNGF) or an antigen-binding fragment thereof. More specifically, the present invention includes pharmaceutical formulations that comprise: (i) a human antibody that specifically binds to hNGF; (ii) a non-ionic surfactant; and (iii) at least one carbohydrate. Specific exemplary components and formulations included within the present invention are described in detail below.

In certain embodiments of the invention, the "surfactant" may be a non-ionic surfactant that is selected from the group consisting of polysorbate 20, polysorbate 80, polyoxyethylene sorbitan monooleate, and polyethylene glycol.

The pharmaceutical formulations of the present invention may, in certain embodiments, be fluid formulations. As used herein, the expression "fluid formulation" means a mixture of at least two components that exists predominantly in the fluid state at about 5°C to about 45°C. Fluid formulations include, *inter alia,* liquid formulations. Fluid formulations may be of low, moderate or high viscosity depending on their particular constituents.

### ANTIBODIES THAT BIND SPECIFICALLY TO hNGF

The pharmaceutical formulations of the present invention may comprise a human antibody, or an antigen-binding fragment thereof, that binds specifically to hNGF. As used herein, the term "hNGF" means a human nerve growth factor having the amino acid sequence as shown in SEQ ID NO: 2, which is encoded by the nucleic acid sequence shown in SEQ ID NO: 1.

The term "antibody", as used herein, is generally intended to refer to immunoglobulin molecules comprising four polypeptide chains, two heavy (H) chains and two light (L) chains inter-connected by disulfide bonds, as well as multimers thereof (e.g., IgM); however, immunoglobulin molecules consisting of only heavy chains (i.e., lacking light chains) are also encompassed within the definition of the term "antibody." Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (CL1). The V_{H} and V_{L} regions can be further subdivided into regions of hypervariability, termed complementary determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4.

Unless specifically indicated otherwise, the term "antibody," as used herein, shall be understood to encompass complete antibody molecules as well as antigen-binding fragments thereof. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. The terms "antigen-binding portion" of an antibody, or "antibody fragment", as used herein, refers to one or more fragments of an antibody that retain the ability to specifically bind to hNGF.

An "isolated antibody", as used herein, is intended to refer to an antibody that is substantially free of other antibodies having different antigenic specificities (e.g., an isolated antibody that specifically binds hNGF is substantially free of antibodies that specifically bind antigens other than hNGF).

The term "specifically binds," or the like, means that an antibody or antigen-binding fragment thereof forms a complex with an antigen that is relatively stable under physiologic conditions. Specific binding can be characterized by a dissociation constant of at least about 1x10⁻⁶ M or greater. Methods for determining whether two molecules specifically bind are well known in the art and include, for example, equilibrium dialysis, surface plasmon resonance, and the like. An isolated antibody that specifically binds hNGF may, however, have cross-reactivity to other antigens, such as NGF molecules from other species. In the context of the present invention, multi-specific (*e.g*., bi-specific) antibodies that bind to hNGF as well as one or more additional antigens are deemed to "specifically bind" hNGF. Moreover, an isolated antibody may be substantially free of other cellular material and/or chemicals.

Exemplary anti-hNGF antibodies that may be included in the pharmaceutical formulations of the present invention are set forth in US 20090041717.

According to certain embodiments of the present invention, the anti-hNGF antibody, or antigen-binding fragment thereof, comprises a heavy chain complementarity determining region (HCDR) 1 having the amino acid sequence of SEQ ID NO: 6, a HCDR2 having the amino acid sequence of SEQ ID NO: 8, and a HCDR3 having the amino acid sequence of SEQ ID NO: 10.

According to certain embodiments of the present invention, the anti-hNGF antibody, or antigen-binding fragment thereof, comprises a light chain complementarity determining region (LCDR) 1 having the amino acid sequence of SEQ ID NO: 14, a LCDR2 having the amino acid sequence of SEQ ID NO:16, and a LCDR3 having the amino acid sequence of SEQ ID NO:18.

In certain embodiments, the anti-hNGF antibody, or antigen-binding fragment thereof, comprises HCDR1-HCDR2-HCDR3 / LCDR1-LCDR2-LCDR3 domains, respectively, selected from the group consisting of SEQ ID NOs:6 - 8 - 10 / SEQ ID NOs:14 - 16 - 18.

In certain embodiments, the anti-hNGF antibody, or antigen-binding fragment thereof, comprises a heavy chain variable region (HCVR) having an amino acid sequence selected from the group consisting of SEQ ID NO:4, and 20. In certain embodiments, the anti-hNGF antibody, or antigen-binding fragment thereof, comprises a light chain variable region (LCVR) having an amino acid sequence selected from the group consisting of SEQ ID NO:12 and 22. In certain embodiments, the anti-hNGF antibody, or antigen-binding fragment thereof, comprises a HCVR/LCVR amino acid sequence pair selected from the group consisting of SEQ ID NO:4/12; and 20/22.

The non-limiting, exemplary antibody used in the Examples herein is referred to as "mAb1." This antibody is also referred to in US 20090041717 and as described herein, comprises an HCVR/LCVR amino acid sequence pair having SEQ ID NOs: 20/22, and HCDR1-HCDR2-HCDR3 / LCDR1-LCDR2-LCDR3 domains represented by SEQ ID NOs:6 - 8 - 10 / SEQ ID NOs:14 - 16 - 18.

The amount of antibody, or antigen-binding fragment thereof, contained within the pharmaceutical formulations of the present invention may vary depending on the specific properties desired of the formulations, as well as the particular circumstances and purposes for which the formulations are intended to be used. In certain embodiments, the pharmaceutical formulations may contain about 0.01 mg/mL to about 500 mg/mL of antibody; about 1 mg/mL to about 500 mg/mL of antibody; about 5 mg/mL to about 400 mg/mL of antibody; about 5 mg/mL to about 200 mg/mL of antibody; about 25 mg/mL to about 180 mg/mL of antibody; about 25 mg/mL to about 150 mg/mL of antibody; or about 50 mg/mL to about 180 mg/mL of antibody. In certain embodiments, the pharmaceutical formulations may contain about 0.1 mg/mL to about 100 mg/mL of antibody; about 0.2 mg/mL to about 75 mg/mL of antibody; about 0.6 mg/mL to about 60 mg/mL of antibody. For example, the formulations of the present invention may comprise about 0.1 mg/mL; about 0.2 mg/mL; about 0.6 mg/mL; about 1 mg/mL; about 2 mg/mL; about 5 mg/mL; about 10 mg/mL; about 15 mg/mL; about 20 mg/mL; about 25 mg/mL; about 30 mg/mL; about 35 mg/mL; about 40 mg/mL; about 45 mg/mL; about 50 mg/mL; about 55 mg/mL; about 60 mg/mL; about 65 mg/mL; about 70 mg/mL; about 75 mg/mL; about 80 mg/mL; about 85 mg/mL; about 86 mg/mL; about 87 mg/mL; about 88 mg/mL; about 89 mg/mL; about 90 mg/mL; about 95 mg/mL; about 100 mg/mL; about 105 mg/mL; about 110 mg/mL; about 115 mg/mL; about 120 mg/mL; about 125 mg/mL; about 130 mg/mL; about 131 mg/mL; about 132 mg/mL; about 133 mg/mL; about 134 mg/mL; about 135 mg/mL; about 140 mg/mL; about 145 mg/mL; about 150 mg/mL; about 155 mg/mL; about 160 mg/mL; about 165 mg/mL; about 170 mg/mL; about 175 mg/mL; about 180 mg/mL; about 185 mg/mL; about 190 mg/mL; about 195 mg/mL; or about 200 mg/mL of an antibody or an antigen-binding fragment thereof, that binds specifically to hNGF.

### EXCIPIENTS and pH

The pharmaceutical formulations of the present invention comprise one or more excipients. The term "excipient," as used herein, means any non-therapeutic agent added to the formulation to provide a desired consistency, viscosity or stabilizing effect.

In certain embodiments, the pharmaceutical formulation of the invention comprises a buffer suitable to maintain a pH ranging from about 4.5 to about 5.6. An exemplary buffer suitable for use in the formulations of the present invention include, e.g. an acetate buffer. In one embodiment, the acetate buffer is prepared at a concentration of 10mM.

The amount of acetate contained within the pharmaceutical formulations of the present invention may vary from about 1 mM to about 50 mM; about 2 mM to about 20 mM; about 3 mM to about 12 mM; or about 10 mM.

The pharmaceutical formulations of the present invention may also comprise one or more carbohydrates, e.g., one or more sugars. The sugar can be a reducing sugar or a non-reducing sugar. "Reducing sugars" include, e.g., sugars with a ketone or aldehyde group and contain a reactive hemiacetal group, which allows the sugar to act as a reducing agent. Specific examples of reducing sugars include fructose, glucose, glyceraldehyde, lactose, arabinose, mannose, xylose, ribose, rhamnose, galactose and maltose. Non-reducing sugars can comprise an anomeric carbon that is an acetal and is not substantially reactive with amino acids or polypeptides to initiate a Maillard reaction. Specific examples of non-reducing sugars include sucrose, trehalose, sorbose, sucralose, sorbitol, melezitose and raffinose. Sugar acids include, for example, saccharic acids, gluconate and other polyhydroxy sugars and salts thereof.

The amount of sugar contained within the pharmaceutical formulations of the present invention will vary depending on the specific circumstances and intended purposes for which the formulations are used. In certain embodiments, the formulations may contain about 0.1% to about 20% sugar; about 0.5% to about 20% sugar; about 1% to about 20% sugar; about 2% to about 15% sugar; about 3% to about 10% sugar; about 4% to about 10% sugar; or about 5% to about 10% sugar. For example, the pharmaceutical formulations of the present invention may comprise about 0.5%; about 1.0%; about 1.5%; about 2.0%; about 2.5%; about 3.0%; about 3.5%; about 4.0%; about 4.5%; about 5.0%; about 5.5%; about 6.0%; 6.5%; about 7.0%; about 7.5%; about 8.0%; about 8.5%; about 9.0%; about 9.5%; about 10.0%; about 10.5%; about 11.0%; about 11.5%; about 12.0%; about 12.5%; about 13.0%; about 13.5%; about 14.0%; about 14.5%; about 15.0%; about 15.5%; about 16.0%; 16.5%; about 17.0%; about 17.5%; about 18.0%; about 18.5%; about 19.0%; about 19.5%; or about 20.0% sugar (e.g., sucrose).

The pharmaceutical formulations of the present invention may also comprise one or more surfactants. As used herein, the term "surfactant" means a substance which reduces the surface tension of a fluid in which it is dissolved and/or reduces the interfacial tension between oil and water. Surfactants can be ionic or non-ionic. Exemplary non-ionic surfactants that can be included in the formulations of the present invention include, e.g., alkyl poly(ethylene oxide), alkyl polyglucosides (e.g., octyl glucoside and decyl maltoside), fatty alcohols such as cetyl alcohol and oleyl alcohol, cocamide MEA, cocamide DEA, and cocamide TEA. Specific non-ionic surfactants that can be included in the formulations of the present invention include, e.g., polysorbates such as polysorbate 20, polysorbate 28, polysorbate 40, polysorbate 60, polysorbate 65, polysorbate 80, polysorbate 81, and polysorbate 85; poloxamers such as poloxamer 188, poloxamer 407; polyethylene-polypropylene glycol; or polyethylene glycol (PEG). Polysorbate 20 is also known as TWEEN 20, sorbitan monolaurate and polyoxyethylenesorbitan monolaurate.

The amount of surfactant contained within the pharmaceutical formulations of the present invention may vary depending on the specific properties desired of the formulations, as well as the particular circumstances and purposes for which the formulations are intended to be used. In certain embodiments, the formulations may contain about 0.01% to about 10% surfactant; about 0.05% to about 5% surfactant; or about 0.1% to about 1% surfactant. For example, the formulations of the present invention may comprise about 0.01%; about 0.02%; about 0.03%; about 0.04%; about 0.05%; about 0.06%; about 0.07%; about 0.08%; about 0.09%; about 0.10%; about 0.11%; about 0.12%; about 0.13%; about 0.14%; about 0.15%; about 0.16%; about 0.17%; about 0.18%; about 0.19%; about 0.20%; about 0.21%; about 0.22%; about 0.23%; about 0.24%; about 0.25%; about 0.26%; about 0.27%; about 0.28%; about 0.29%; or about 0.30% surfactant (e.g., polysorbate 20).

The pharmaceutical formulations of the present invention may have a pH of from about 4.0 to about 6.0. For example, the formulations of the present invention may have a pH of about 4.2; about 4.4; about 4.6; about 4.8; about 5.0; about 5.2; about 5.4; about 5.6; about 5.8; or about 6.0.

### EXEMPLARY FORMULATIONS

According to one aspect of the present invention, the pharmaceutical formulation comprises: (i) a human antibody that specifically binds to hNGF (e.g., mAb1); (ii) acetate; and (iii) a sugar (*e.g*., sucrose). Specific, non-limiting exemplary embodiments encompassed by this aspect of the invention are set forth in Table 1.

**Table 1**

| **Exemplary Pharmaceutical Formulations Comprising mAb1, Acetate and Sucrose** | | | | | | | | | | | | | | | | |
|---|---|---|---|---|---|---|---|---|---|---|---|---|---|---|---|---|
| mAb1 (mg/ml) | 5 | 50 | 100 | 150 | 5 | 50 | 100 | 150 | 5 | 50 | 100 | 150 | 5 | 50 | 100 | 150 |
| acetate (mM) | 10 | 10 | 10 | 10 | 10 | 10 | 10 | 10 | 10 | 10 | 10 | 10 | 10 | 10 | 10 | 10 |
| sucrose (%) | 2 | 2 | 2 | 2 | 4 | 4 | 4 | 4 | 6 | 6 | 6 | 6 | 8 | 8 | 8 | 8 |

According to another aspect of the present invention, the pharmaceutical formulation comprises: (i) a human antibody that specifically binds to hNGF (*e.g*., mAb1); (ii) acetate; (iii) a sugar (*e.g*., sucrose); and (iv) a surfactant (*e.g*., polysorbate 20). Specific, non-limiting exemplary embodiments encompassed by this aspect of the invention are set forth in Tables 2A and 2B.

**Table 2A**

| **Exemplary Pharmaceutical Formulations Comprising mAb1, Acetate, Sucrose and Polysorbate 20** | | | | | | | | | | | | |
|---|---|---|---|---|---|---|---|---|---|---|---|---|
| mAb1 (mg/ml) | 5 | 50 | 100 | 150 | 5 | 50 | 100 | 150 | 5 | 50 | 100 | 150 |
| acetate (mM) | 10 | 10 | 10 | 10 | 10 | 10 | 10 | 10 | 10 | 10 | 10 | 10 |
| sucrose (%) | 2 | 2 | 2 | 2 | 4 | 4 | 4 | 4 | 8 | 8 | 8 | 8 |
| polysorbate 20 (%) | 0.05 | 0.05 | 0.05 | 0.05 | 0.05 | 0.05 | 0.05 | 0.05 | 0.05 | 0.05 | 0.05 | 0.05 |

**Table 2B**

| **Exemplary Pharmaceutical Formulations Comprising mAb1, Acetate, Sucrose and Polysorbate 20** | | | | | | | | | | | | |
|---|---|---|---|---|---|---|---|---|---|---|---|---|
| mAb1 (mg/ml) | 5 | 50 | 100 | 150 | 5 | 50 | 100 | 150 | 5 | 50 | 100 | 150 |
| acetate (mM) | 10 | 10 | 10 | 10 | 10 | 10 | 10 | 10 | 10 | 10 | 10 | 10 |
| sucrose (%) | 2 | 2 | 2 | 2 | 4 | 4 | 4 | 4 | 8 | 8 | 8 | 8 |
| polysorbate 20 (%) | 0.1 | 0.1 | 0.1 | 0.1 | 0.1 | 0.1 | 0.1 | 0.1 | 0.1 | 0.1 | 0.1 | 0.1 |

According to another aspect of the present invention, the pharmaceutical formulation comprises: (i) a human antibody that specifically binds to hNGF (e.g., mAb1); (ii) acetate; (iii) a sugar (e*.g.,* sucrose); (iv) a surfactant (*e.g*., polysorbate 20); (v) a first amino acid (*e.g.,* histidine) and (v) a second amino acid (*e.g.,* arginine). Specific, non-limiting exemplary embodiments encompassed by this aspect of the invention are set forth in Tables 3A, 3B, 3C, 3D, 3E and 3F.

**Table 3A**

| **Exemplary Pharmaceutical Formulations Comprising mAb1, Acetate, Histidine, Sucrose, Polysorbate 20 and Arginine** | | | | | | | | | | | | |
|---|---|---|---|---|---|---|---|---|---|---|---|---|
| mAb1 (mg/ml) | 5 | 50 | 100 | 150 | 5 | 50 | 100 | 150 | 5 | 50 | 100 | 150 |
| Acetate (mM) | 10 | 10 | 10 | 10 | 10 | 10 | 10 | 10 | 10 | 10 | 10 | 10 |
| histidine (mM) | 10 | 10 | 10 | 10 | 10 | 10 | 10 | 10 | 10 | 10 | 10 | 10 |
| sucrose (%) | 2 | 2 | 2 | 2 | 4 | 4 | 4 | 4 | 8 | 8 | 8 | 8 |
| polysorbate 20 (%) | 0.05 | 0.05 | 0.05 | 0.05 | 0.05 | 0.05 | 0.05 | 0.05 | 0.05 | 0.05 | 0.05 | 0.05 |
| arginine (mM) | 10 | 10 | 10 | 10 | 10 | 10 | 10 | 10 | 10 | 10 | 10 | 10 |

**Table 3B**

| **Exemplary Pharmaceutical Formulations Comprising mAb1, Acetate, Histidine, Sucrose, Polysorbate 20 and Arginine** | | | | | | | | | | | | |
|---|---|---|---|---|---|---|---|---|---|---|---|---|
| mAb1 (mg/ml) | 5 | 50 | 100 | 150 | 5 | 50 | 100 | 150 | 5 | 50 | 100 | 150 |
| Acetate (mM) | 10 | 10 | 10 | 10 | 10 | 10 | 10 | 10 | 10 | 10 | 10 | 10 |
| histidine (mM) | 10 | 10 | 10 | 10 | 10 | 10 | 10 | 10 | 10 | 10 | 10 | 10 |
| sucrose (%) | 2 | 2 | 2 | 2 | 4 | 4 | 4 | 4 | 8 | 8 | 8 | 8 |
| polysorbate 20 (%) | 0.05 | 0.05 | 0.05 | 0.05 | 0.05 | 0.05 | 0.05 | 0.05 | 0.05 | 0.05 | 0.05 | 0.05 |
| arginine (mM) | 25 | 25 | 25 | 25 | 25 | 25 | 25 | 25 | 25 | 25 | 25 | 25 |

**Table 3C**

| **Exemplary Pharmaceutical Formulations Comprising mAb1, Acetate, Histidine, Sucrose, Polysorbate 20 and Arginine** | | | | | | | | | | | | |
|---|---|---|---|---|---|---|---|---|---|---|---|---|
| mAb1 (mg/ml) | 5 | 50 | 100 | 150 | 5 | 50 | 100 | 150 | 5 | 50 | 100 | 150 |
| Acetate (mM) | 10 | 10 | 10 | 10 | 10 | 10 | 10 | 10 | 10 | 10 | 10 | 10 |
| histidine (mM) | 25 | 25 | 25 | 25 | 25 | 25 | 25 | 25 | 25 | 25 | 25 | 25 |
| sucrose (%) | 2 | 2 | 2 | 2 | 4 | 4 | 4 | 4 | 8 | 8 | 8 | 8 |
| polysorbate 20 (%) | 0.05 | 0.05 | 0.05 | 0.05 | 0.05 | 0.05 | 0.05 | 0.05 | 0.05 | 0.05 | 0.05 | 0.05 |
| arginine (mM) | 10 | 10 | 10 | 10 | 10 | 10 | 10 | 10 | 10 | 10 | 10 | 10 |

**Table 3D**

| **Exemplary Pharmaceutical Formulations Comprising mAb1, Acetate, Histidine, Sucrose, Polysorbate 20 and Arginine** | | | | | | | | | | | | |
|---|---|---|---|---|---|---|---|---|---|---|---|---|
| mAb1 (mg/ml) | 5 | 50 | 100 | 150 | 5 | 50 | 100 | 150 | 5 | 50 | 100 | 150 |
| Acetate (mM) | 10 | 10 | 10 | 10 | 10 | 10 | 10 | 10 | 10 | 10 | 10 | 10 |
| histidine (mM) | 25 | 25 | 25 | 25 | 25 | 25 | 25 | 25 | 25 | 25 | 25 | 25 |
| sucrose (%) | 2 | 2 | 2 | 2 | 4 | 4 | 4 | 4 | 8 | 8 | 8 | 8 |
| polysorbate 20 (%) | 0.05 | 0.05 | 0.05 | 0.05 | 0.05 | 0.05 | 0.05 | 0.05 | 0.05 | 0.05 | 0.05 | 0.05 |
| arginine (mM) | 25 | 25 | 25 | 25 | 25 | 25 | 25 | 25 | 25 | 25 | 25 | 25 |

**Table 3E**

| **Exemplary Pharmaceutical Formulations Comprising mAb1, Acetate, Histidine, Sucrose, Polysorbate 20 and Arginine** | | | | | | | | | | | | |
|---|---|---|---|---|---|---|---|---|---|---|---|---|
| mAb1 (mg/ml) | 5 | 50 | 100 | 150 | 5 | 50 | 100 | 150 | 5 | 50 | 100 | 150 |
| Acetate (mM) | 10 | 10 | 10 | 10 | 10 | 10 | 10 | 10 | 10 | 10 | 10 | 10 |
| histidine (mM) | 25 | 25 | 25 | 25 | 25 | 25 | 25 | 25 | 25 | 25 | 25 | 25 |
| sucrose (%) | 2 | 2 | 2 | 2 | 5 | 5 | 5 | 5 | 10 | 10 | 10 | 10 |
| polysorbate 20 (%) | 0.2 | 0.2 | 0.2 | 0.2 | 0.2 | 0.2 | 0.2 | 0.2 | 0.2 | 0.2 | 0.2 | 0.2 |
| arginine (mM) | 50 | 50 | 50 | 50 | 50 | 50 | 50 | 50 | 50 | 50 | 50 | 50 |

**Table 3F**

| **Exemplary Pharmaceutical Formulations Comprising mAb1, Acetate, Histidine, Sucrose, Polysorbate 20 and Arginine** | | | | | | | | | | | | |
|---|---|---|---|---|---|---|---|---|---|---|---|---|
| mAb1 (mg/ml) | 5 | 50 | 100 | 150 | 5 | 50 | 100 | 150 | 5 | 50 | 100 | 150 |
| Acetate (mM) | 10 | 10 | 10 | 10 | 10 | 10 | 10 | 10 | 10 | 10 | 10 | 10 |
| histidine (mM) | 25 | 25 | 25 | 25 | 25 | 25 | 25 | 25 | 25 | 25 | 25 | 25 |
| sucrose (%) | 2 | 2 | 2 | 2 | 5 | 5 | 5 | 5 | 10 | 10 | 10 | 10 |
| polysorbate 20 (%) | 0.2 | 0.2 | 0.2 | 0.2 | 0.2 | 0.2 | 0.2 | 0.2 | 0.2 | 0.2 | 0.2 | 0.2 |
| arginine (mM) | 25 | 25 | 25 | 25 | 25 | 25 | 25 | 25 | 25 | 25 | 25 | 25 |

According to certain aspects of the invention, the antibody formulation may comprise an anti-hNGF antibody at a concentration of about 20 mg/mL in about 10 mM acetate, plus about 1.0% Polyethylene glycol 3350 (PEG 3350), and about 20% sucrose at a pH of about 5.0. In one embodiment, the antibody formulation is administered intravenously. In one embodiment, the antibody formulation is administered subcutaneously.

Additional non-limiting examples of pharmaceutical formulations encompassed by the present invention are set forth elsewhere herein, including the working Examples presented below.

### STABILITY AND VISCOSITY OF THE PHARMACEUTICAL FORMULATIONS

The pharmaceutical formulations of the present invention typically exhibit high levels of stability. The term "stable," as used herein in reference to the pharmaceutical formulations, means that the antibodies within the pharmaceutical formulations retain an acceptable degree of structure and/or function and/or biological activity after storage for a defined amount of time. A formulation may be stable even though the antibody contained therein does not maintain 100% of its structure and/or function and/or biological activity after storage for a defined amount of time. Under certain circumstances, maintenance of about 80%, about 85%, about 90%, about 95%, about 96%, about 97%, about 98% or about 99% of an antibody's structure and/or function and/or biological activity after storage for a defined amount of time may be regarded as "stable."

Stability can be measured, *inter alia,* by determining the percentage of native antibody remaining in the formulation after storage for a defined amount of time at a given temperature. The percentage of native antibody can be determined by, *inter alia,* size exclusion chromatography (e.g., size exclusion high performance liquid chromatography [SE-HPLC]). An "acceptable degree of stability," as that phrase is used herein, means that at least 90% of the native form of the antibody can be detected in the formulation after storage for a defined amount of time at a given temperature. In certain embodiments, at least about 90%, 91%, 92%, 93%, 94%, 95%, 96%, 97%, 98%, 99% or 100% of the native form of the antibody can be detected in the formulation after storage for a defined amount of time at a given temperature. The defined amount of time after which stability is measured can be at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or more. The temperature at which the pharmaceutical formulation may be stored when assessing stability can be any temperature from about -80°C to about 45°C, e.g., storage at about -30°C, about -20°C, about 0°C, about 5°C, about 25°C, about 37°C, or about 45°C. For example, a pharmaceutical formulation may be deemed stable if after 3 months of storage at 5°C, greater than about 90%, 95%, 96% or 97% of native antibody is detected by SE-HPLC. A pharmaceutical formulation may also be deemed stable if after 6 months of storage at 5°C, greater than about 90%, 95%, 96% or 97% of native antibody is detected by SE-HPLC. A pharmaceutical formulation may also be deemed stable if after 9 months of storage at 5°C, greater than about 90%, 95%, 96% or 97% of native antibody is detected by SE-HPLC. A pharmaceutical formulation may also be deemed stable if after 3 months of storage at 25°C, greater than about 90%, 95%, 96% or 97% of native antibody is detected by SE-HPLC. A pharmaceutical formulation may also be deemed stable if after 6 months of storage at 25°C, greater than about 90%, 95%, 96% or 97% of native antibody is detected by SE-HPLC. A pharmaceutical formulation may also be deemed stable if after 9 months of storage at 25°C, greater than about 90%, 95%, 96% or 97% of native antibody is detected by SE-HPLC.

Other methods may be used to assess the stability of the formulations of the present invention such as, e.g., differential scanning calorimetry (DSC) to determine thermal stability, controlled agitation to determine mechanical stability, and absorbance at about 350 nm or about 405 nm to determine solution turbidities. For example, a formulation of the present invention may be considered stable if, after 6 or more months of storage at about 5°C to about 25°C, the change in OD₄₀₅ of the formulation is less than about 0.05 (e.g., 0.04, 0.03, 0.02, 0.01, or less) from the OD₄₀₅ of the formulation at t=0.

Stability may also be assessed by measuring the biological activity and/or binding affinity of the antibody to its target. For example, a formulation of the present invention may be regarded as stable if, after storage at e.g., 5°C, 25°C, 37°C, 45°C, etc. for a defined amount of time (e.g., 1 to 12 or 18 months), the anti-hNGF antibody contained within the formulation binds to hNGF with an affinity that is at least 50%, 60%, 70%, 80%, 90%, 95%, or more of the binding affinity of the antibody prior to said storage. Additional methods for assessing the stability of an antibody in formulation are demonstrated in the Examples presented below.

In the fluid form, the pharmaceutical formulations of the present invention may, in certain embodiments, exhibit low to moderate levels of viscosity. "Viscosity" as used herein may be "kinematic viscosity" or "absolute viscosity." "Kinematic viscosity" is a measure of the resistive flow of a fluid under the influence of gravity. When two fluids of equal volume are placed in identical capillary viscometers and allowed to flow by gravity, a viscous fluid takes longer than a less viscous fluid to flow through the capillary. For example, if one fluid takes 200 seconds to complete its flow and another fluid takes 400 seconds, the second fluid is twice as viscous as the first on a kinematic viscosity scale. "Absolute viscosity", sometimes called dynamic or simple viscosity, is the product of kinematic viscosity and fluid density (Absolute Viscosity = Kinematic Viscosity x Density). The dimension of kinematic viscosity is L²/T where L is a length and T is a time. Commonly, kinematic viscosity is expressed in centistokes (cSt). The SI unit of kinematic viscosity is mm²/s, which is 1 cSt. Absolute viscosity is expressed in units of centipoise (cP). The SI unit of absolute viscosity is the milliPascal-second (mPa-s), where 1 cP = 1 mPa-s.

As used herein, a low level of viscosity, in reference to a fluid formulation of the present invention, will exhibit an absolute viscosity of less than about 20 cPoise (cP). For example, a fluid formulation of the invention will be deemed to have "low viscosity," if, when measured using standard viscosity measurement techniques, the formulation exhibits an absolute viscosity of about 19 cP, about 18 cP, about 17 cP, about 16 cP, about 15 cP, about 14 cP, about 13 cP, about 12 cP, about 11 cP, about 10 cP, about 9 cP, about 8 cP, about 7 cP, about 6 cP, about 5 cP, about 4 cP, or less. As used herein, a moderate level of viscosity, in reference to a fluid formulation of the present invention, will exhibit an absolute viscosity of between about 30 cP and about 20 cP. For example, a fluid formulation of the invention will be deemed to have "moderate viscosity," if when measured using standard viscosity measurement techniques, the formulation exhibits an absolute viscosity of about 30 cP, about 29 cP, about 28 cP, about 27 cP, about 26 cP, about 25 cP, about 24 cP, about 23 cP, about 22 cP, about 21 cP or about 20 cP.

As illustrated in Example 5 below, the present inventors have made the surprising discovery that low to moderate viscosity fluid formulations comprising high concentrations of an anti-hNGF antibody (e.g., up to at least 125 to 150 mg/mL) can be obtained by formulating the antibody with 25 mM histidine and 25 mM to 100 mM arginine. In addition, it was further discovered that the viscosity of the formulation could be decreased to an even greater extent by lowering the sucrose content.

### CONTAINERS FOR THE PHARMACEUTICAL FORMULATIONS AND METHODS OF ADMINISTRATION

The pharmaceutical formulations of the present invention may be contained within any container suitable for storage of medicines and other therapeutic compositions. For example, the pharmaceutical formulations may be contained within a sealed and sterilized plastic or glass container having a defined volume such as a vial, ampule, syringe, cartridge, or bottle. Different types of vials can be used to contain the formulations of the present invention including, e.g., clear and opaque (*e.g.,* amber) glass or plastic vials. Likewise, any type of syringe can be used to contain and/or administer the pharmaceutical formulations of the present invention. The pharmaceutical formulation within the container may be treated using any method known in the art to remove oxygen to improve antibody stability if necessary. The oxygen in the headspace (the gaseous space above a liquid in a closed container) may be replaced by an inert gas, such as nitrogen or argon.

The pharmaceutical formulations of the present invention may be contained within "normal tungsten" syringes or "low tungsten" syringes. As will be appreciated by persons of ordinary skill in the art, the process of making glass syringes generally involves the use of a hot tungsten rod which functions to pierce the glass thereby creating a hole from which liquids can be drawn and expelled from the syringe. This process results in the deposition of trace amounts of tungsten on the interior surface of the syringe. Subsequent washing and other processing steps can be used to reduce the amount of tungsten in the syringe. As used herein, the term "normal tungsten" means that the syringe contains greater than 500 parts per billion (ppb) of tungsten. The term "low tungsten" means that the syringe contains less than 500 ppb of tungsten. For example, a low tungsten syringe, according to the present invention, can contain less than about 490, 480, 470, 460, 450, 440, 430, 420, 410, 390, 350, 300, 250, 200, 150, 100, 90, 80, 70, 60, 50, 40, 30, 20, 10 or fewer ppb of tungsten.

The rubber plungers used in syringes, and the rubber stoppers used to close the openings of vials, may be coated to prevent contamination of the medicinal contents of the syringe or vial and/or to preserve their stability. Thus, pharmaceutical formulations of the present invention, according to certain embodiments, may be contained within a syringe that comprises a coated plunger, or within a vial that is sealed with a coated rubber stopper. For example, the plunger or stopper may be coated with a fluorocarbon film. Examples of coated stoppers and/or plungers suitable for use with vials and syringes containing the pharmaceutical formulations of the present invention are mentioned in, e.g., U.S. Patent Nos. 4,997,423; 5,908,686; 6,286,699; 6,645,635; and 7,226,554. Particular exemplary coated rubber stoppers and plungers that can be used in the context of the present invention are commercially available under the tradename "FluroTec®," available from West Pharmaceutical Services, Inc. (Lionville, PA).

According to certain embodiments of the present invention, the pharmaceutical formulations may be contained within a low tungsten syringe that comprises a fluorocarbon-coated plunger. However, as demonstrated in the Examples section below, the anti-NGF antibody of the invention appears to be stable in any of the combinations of syringe and plunger tested.

The pharmaceutical formulations can be administered to a patient by parenteral routes such as injection (*e.g.,* subcutaneous, intravenous, intramuscular, intraperitoneal, etc.) or percutaneous, mucosal, nasal, pulmonary and/or oral administration. Numerous reusable pen and/or autoinjector delivery devices can be used to subcutaneously deliver the pharmaceutical formulations of the present invention. Examples include, but are not limited to AUTOPEN™ (Owen Mumford, Inc., Woodstock, UK), DISETRONlC™ pen (Disetronic Medical Systems, Bergdorf, Switzerland), HUMALOG MIX 75/25™ pen, HUMALOG™ pen, HUMALIN 70/30™ pen (Eli Lilly and Co., Indianapolis, IN), NOVOPEN™ I, II and III (Novo Nordisk, Copenhagen, Denmark), NOVOPEN JUNIOR™ (Novo Nordisk, Copenhagen, Denmark), BD™ pen (Becton Dickinson, Franklin Lakes, NJ), OPTIPEN™, OPTIPEN PRO™, OPTIPEN STARLET™, and OPTICLIK™ (Sanofi-Aventis, Frankfurt, Germany), to name only a few. Examples of disposable pen and/or autoinjector delivery devices having applications in subcutaneous delivery of a pharmaceutical composition of the present invention include, but are not limited to the SOLOSTAR™ pen (Sanofi-Aventis), the FLEXPEN™ (Novo Nordisk), and the KWIKPEN™ (Eli Lilly), the SURECLICK™ Autoinjector (Amgen, Thousand Oaks, CA), the PUSHCLICK™ (Scandinavian Health, Ltd. (SHL) Group), the PENLET™ (Haselmeier, Stuttgart, Germany), the EPIPEN (Dey, L.P.), and the HUMIRA™ ^{P}en (Abbott Labs, Abbott Park, IL), to name only a few.

The use of a microinfusor to deliver the pharmaceutical formulations of the present invention is also contemplated herein. As used herein, the term "microinfusor" means a subcutaneous delivery device designed to slowly administer large volumes (*e.g.,* up to about 2.5 mL or more) of a therapeutic formulation over a prolonged period of time (*e.g.,* about 10, 15, 20, 25, 30 or more minutes). *See, e.g.,* U.S. 6,629,949; US 6,659,982; and Meehan et al., J. Controlled Release 46:107-116 (1996). Microinfusors are particularly useful for the delivery of large doses of therapeutic proteins contained within high concentration (e.g., about 100, 125, 150, 175, 200 or more mg/mL) and/or viscous solutions.

### THERAPEUTIC USES OF THE PHARMACEUTICAL FORMULATIONS

The pharmaceutical formulations of the present invention are useful, *inter alia,* for the treatment, prevention and/or amelioration of any disease or disorder associated with NGF activity. Exemplary, non-limiting diseases and disorders that can be treated and/or prevented by the administration of the pharmaceutical formulations of the present invention include, pain resulting from any condition associated with neurogenic, neuropathic or nociceptic pain. In certain embodiments of neuropathic pain, referred trigeminal neuralgia, post-herpetic neuralgia, phantom limb pain, fibromyalgia, reflex sympathetic dystrophy and neurogenic pain conditions are preferably treated. In other embodiments, cancer pain, particularly, bone cancer pain, osteoarthritis or rheumatoid arthritis pain, lower back pain, post-operative incision pain, fracture pain, osteoporotic fracture pain, osteoporosis, gout joint pain, diabetic neuropathy, sciatica, pains associated with sickle cell crises, migraine, and other neuropathic and/or nociceptic pain are preferably treated. Thus, the present invention includes methods of treating, preventing, and/or ameliorating any disease or disorder associated with NGF activity or NGF activation (including any of the above mentioned exemplary diseases, disorders and conditions) through use of the pharmaceutical formulations of the invention. The therapeutic methods of the present invention comprise administering to a subject any formulation comprising an anti-hNGF antibody as disclosed herein. The subject to which the pharmaceutical formulation is administered can be, e.g., any human or non-human animal that is in need of such treatment, prevention and/or amelioration, or who would otherwise benefit from the inhibition or attenuation of NGF and/or NGF-mediated activity. For example, the subject can be an individual that is diagnosed with, or who is deemed to be at risk of being afflicted by any of the aforementioned diseases or disorders. The present invention further includes the use of any of the pharmaceutical formulations disclosed herein in the manufacture of a medicament for the treatment, prevention and/or amelioration of any disease or disorder associated with NGF activity or NGF activation (including any of the above mentioned exemplary diseases, disorders and conditions).

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Stability of a Fully Human Anti-Human Nerve Growth Factor (NGF) Antibody ("mAb1") After Storage at Low Temperatures

In this Example, various formulations were created containing an anti-human NGF antibody without excipients. The exemplary antibody used in this and all subsequent Examples set forth below is an antibody comprising a heavy chain variable region (HCVR) with the amino acid sequence of SEQ ID NO:20, and a light chain variable region (LCVR) with the amino acid sequence of SEQ ID NO:22. This antibody is referred to herein as "mAb1".

As a preliminary experiment, the stability of mAb1 in liquid solution was determined following various amounts of time in frozen storage at -30°C and -80°C. The concentration of mAb1 used in this Example was 130 mg/mL. At various time points, the stability of mAb1 was determined by size exclusion high performance liquid chromatography (SE-HPLC) and by cation exchange high performance liquid chromatography (CEX-HPLC). Stability was assessed based on the percentage of native mAb1 remaining in the sample (by SE-HPLC; Table 4) and by the percentage of acidic species observed in the sample (by CEX-HPLC; Table 5). An increase in percent acidic species is consistent with deamidation of the antibody and is thus considered an undesired phenomenon with respect to the pharmaceutical formulations of the present invention.

**Table 4**

| **% Native mAb Remaining (SE-HPLC)** | | |
|---|---|---|
| | **Storage Temperature** | |
| **Time(months)** | **-80°C** | **-30°C** |
| 0 | 96.6 | 96.6 |
| 1 | 96.6 | 95.2 |
| 3 | 96.6 | 94.6 |
| 6 | 96.6 | 94.2 |
| 9 | 96.5 | 93.1 |
| 12 | 96.7 | 93.3 |

**Table 5**

| **% Acidic Species (CEX-HPLC)** | | |
|---|---|---|
| | **Storage Temperature** | |
| **Time(months)** | **-80°C** | **-30°C** |
| 0 | 14.3 | 14.3 |
| 1 | 14.7 | 14.6 |
| 3 | 14.9 | 14.5 |
| 6 | 14.1 | 12.8 |
| 9 | 14.9 | 14.5 |
| 12 | 14.6 | 14.6 |

These results show that mAb1 can remain stable at a concentration of 130 mg/mL for at least 12 months when stored at -80°C.

### Example 2. Stability of mAb1 Formulations Containing Minimal Excipients

Six different formulations containing mAb1 were prepared at a concentration of 40 mg/mL with minimal excipients (as shown in Table 6, see also Example 2) and were stored at - 20°C or -30°C for various periods of time. All formulations contain 10 mM acetate, pH 5.0. Table 7 (-30°C ) and Table 8 (-20°C) show the percent of native mAb1 remaining in various minimal excipient formulations, as measured by SE-HPLC.

**Table 6**

| **mAb1 Minimal Excipient Formulations** | | |
|---|---|---|
| **Formulation** | **Excipient** | **mAb1 (mg/mL)** |
| 1 | 0.5% polyethylene glycol 3350 | 40 |
| 2 | 1.0% polyethylene glycol 3350 | 40 |
| 3 | 1% sucrose | 40 |
| 4 | 2% sucrose | 40 |
| 5 | 4% sucrose | 40 |
| 6 | none | 40 |

As noted above, the formulations were tested for stability by SE-HPLC after various amounts of time at -30°C and -20°C. The results, expressed in percent of native mAb1 remaining, are shown in Tables 7 (-30°C storage) and 8 (-20°C).

**Table 7**

| **% Native mAb1 Remaining (SE-HPLC) After Storage at -30°C** | | | | | | |
|---|---|---|---|---|---|---|
| | **Formulation # (see Table 6)** | | | | | |
| **Time (months)** | **1** | **2** | **3** | **4** | **5** | **6** |
| 0 | 98.3 | 98.1 | 98.0 | 97.9 | 98.1 | 98.0 |
| 1 | 98.0 | 98.1 | 98.0 | 98.0 | 98.0 | 97.0 |
| 3 | 97.8 | 97.9 | 98.0 | 98.0 | 97.9 | 96.0 |
| 6 | 98.2 | 98.4 | 98.5 | 98.4 | 98.4 | 95.9 |
| 9 | 98.2 | 98.3 | 98.5 | 98.5 | 98.8 | 95.1 |
| 12 | 98.2 | 98.4 | 98.4 | 98.4 | 98.5 | 95.7 |
| 18 | 97.7 | 98.2 | 98.1 | 98.1 | 98.4 | 94.5 |
| 24 | 97.9 | 98.1 | 98.3 | 98.5 | 98.5 | 94.5 |

**Table 8**

| **% Native mAb1 Remaining (SE-HPLC) After Storage at -20°C** | | | | | | |
|---|---|---|---|---|---|---|
| | **Formulation # (see Table 6)** | | | | | |
| **Time (months)** | **1** | **2** | **3** | **4** | **5** | **6** |
| 0 | 98.3 | 98.1 | 98.0 | 97.9 | 98.1 | 98.0 |
| 1 | 97.8 | 98.1 | 98.2 | 98.1 | 98.2 | 95.8 |
| 3 | 97.8 | 97.9 | 98.1 | 98.2 | 98.0 | 93.0 |
| 6 | 97.7 | 98.3 | 98.3 | 98.4 | 98.5 | 92.6 |
| 9 | 97.8 | 98.1 | 98.3 | 98.3 | 98.3 | 90.7 |
| 12 | 97.7 | 98.2 | 98.3 | 98.3 | 98.5 | 92.9 |
| 18 | 96.4 | 97.7 | 97.9 | 98.1 | 98.3 | 86.7 |
| 24 | 96.9 | 97.9 | 98.1 | 98.2 | 98.3 | 88.6 |

As shown in this Example, the stability of mAb1 was maintained to a significant extent in formulations 1, 2, 3, 4, and 5 after several months of storage at -20°C and -30°C. These results indicate that the stability of mAb1 at -30°C can be enhanced by the addition of at least 1.0% sucrose, or at least 0.5% polyethylene glycol 3350. These results further indicate that the stability of mAb1 at -20°C can be enhanced by the addition of at least 1.0% sucrose, or at least 1.0% polyethylene glycol 3350.

### Example 3. Stabilized Formulation of mAb1

A stabilized formulation containing various concentrations of mAb1 was prepared for use in Examples 4 and 5 below. This formulation, designated "Formulation A", is shown in Table 9.

**Table 9**

| **Stabilized mAb1 Formulation "A"** | |
|---|---|
| **Component** | **Formulation A** |
| mAb1 | 6 - 100 mg/mL |
| Acetate | 10 mM |
| Polysorbate 20 | 0.05% |
| Sucrose | 8% |
| pH | 5.0 |

### Example 4. Stability of Formulation A After Storage at 5°C

Formulation A (see Example 3) containing 6, 20 or 100 mg/mL mAb1 was tested for stability after several months of storage at 5°C in clear glass vials. Stability was assessed by the following parameters: (a) visual appearance; (b) turbidity (OD 405 nm); (c) pH; (d) percent total mAb1 recovered as measured by RP-HPLC; (e) percent native mAb1 recovered (as measured by SE-HPLC); (f) percent main peak mAb1 recovered (as measured by CEX-HPLC); and (g) percent acidic species mAb1 recovered (as measured by CEX-HPLC). The stability results for Formulation A containing 6, 20 and 100 mg/mL of mAb1 are summarized in Tables 10, 11 and 12, respectively.

**Table 10**

| **Stability of Formulation A Containing 6 mg/mL mAb1 After Storage at 5°C in Glass Vials (0-12 months)** | | | | | | | |
|---|---|---|---|---|---|---|---|
| | **Length of 5°C Storage (months)** | | | | | | |
| **Parameter** | **0** | **1** | **2** | **3** | **6** | **9** | **12** |
| Visual Appearance | Pass | Pass | Pass | Pass | Pass | Pass | Pass |
| Turbidity (OD 405 nm) | 0.00 | 0.00 | 0.00 | 0.00 | 0.00 | 0.00 | 0.00 |
| pH | 5.1 | 5.1 | 5.1 | 5.0 | 5.0 | 5.1 | 5.1 |
| % Total mAb1 Recovered | 100 | 96 | 101 | 99 | 102 | 107 | 107 |
| % Native mAb1 Recovered (SE-HPLC) | 98.8 | 98.6 | 98.7 | 98.9 | 98.5 | 98.8 | 98.7 |
| % Main Peak mAb1 Recovered (CEX-HPLC) | 44.8 | 46.3 | 46.4 | 45.4 | 41.2 | 46.2 | 45.0 |
| % Acidic Species mAb1 Recovered (CEX-HPLC) | 16.6 | 16.5 | 17.4 | 17.7 | 15.2 | 15.9 | 16.5 |

**Table 11**

| **Stability of Formulation A Containing 20 mg/mL mAb1 After Storage at 5°C in Glass Vials (0-18 months)** | | | | | | | | |
|---|---|---|---|---|---|---|---|---|
| **Length** of **5°C Storage (months)** | | | | | | | | |
| **Parameter** | **0** | **1** | **2** | **3** | **6** | **9** | **12** | **18** |
| Visual Appearance | Pass | Pass | Pass | Pass | Pass | Pass | Pass | Pass |
| Turbidity (OD 405 nm) | 0.00 | 0.00 | 0.01 | 0.00 | 0.00 | 0.00 | 0.00 | 0.00 |
| pH | 5.1 | 5.2 | 5.0 | 5.1 | 5.0 | 5.1 | 5.2 | 5.1 |
| % Total mAb1 Recovered | 100 | 96 | 99 | 100 | 100 | 104 | 101 | 102 |
| % Native mAb1 Recovered (SE-HPLC) | 99.0 | 98.4 | 98.5 | 98.4 | 98.6 | 98.2 | 98.6 | 98.2 |
| % Main Peak mAb1 Recovered (CEX-HPLC) | 44.0 | 44.0 | 44.1 | 43.8 | 44.3 | 43.8 | 44.9 | 43.6 |
| % Acidic Species mAb1 Recovered (CEX-HPLC) | 15.8 | 15.5 | 15.8 | 15.5 | 15.5 | 15.2 | 15.9 | 15.3 |

**Table 12**

| **Stability of Formulation A Containing 100 mg/mL mAb1 After Storage at 5°C in Glass Vials (0-18 months)** | | | | | | | | |
|---|---|---|---|---|---|---|---|---|
| **Length of 5°C Storage (months)** | | | | | | | | |
| **Parameter** | **0** | **1** | **2** | **3** | **6** | **9** | **12** | **18** |
| Visual Appearance | Pass | Pass | Pass | Pass | Pass | Pass | Pass | Pass |
| Turbidity (OD 405 nm) | 0.00 | 0.00 | 0.00 | 0.00 | 0.00 | 0.00 | 0.00 | 0.00 |
| pH | 5.0 | 5.1 | 5.0 | 5.1 | 5.0 | 5.1 | 5.0 | 5.2 |
| % Total mAb1 Recovered | 100 | 98 | 106 | 104 | 103 | 103 | 101 | 104 |
| % Native mAb1 Recovered (SE-HPLC) | 98.2 | 97.9 | 97.5 | 97.8 | 98.2 | 97.4 | 98.1 | 97.6 |
| % Main Peak mAb1 Recovered (CEX-HPLC) | 41.3 | 40.9 | 39.2 | 39.0 | 39.5 | 39.7 | 42.0 | 40.4 |
| % Acidic Species mAb1 Recovered (CEX-HPLC) | 14.8 | 14.9 | 13.7 | 14.0 | 14.0 | 13.4 | 13.8 | 14.0 |

The results of this Example demonstrate that Formulation A containing 6 mg/mL mAb1 remained stable for at least 12 months of storage, at 5°C in clear glass vials, with about 98.7% or more of native mAb1 remaining in the samples after 12 months of storage under such conditions. The results of this Example further demonstrate that Formulation A containing 20 mg/mL mAb1 remained stable for at least 18 months of storage, at 5°C in clear glass vials, with about 98.2% of native mAb1 remaining in the samples after 18 months of storage under such conditions. In addition, Formulation A containing 100 mg/mL mAb1 remained stable for at least 18 months of storage, at 5°C in clear glass vials, with about 97.6% of native mAb1 remaining in the samples after 18 months of storage under such conditions. In addition, the percent acidic species did not change significantly from time 0 after 12 or 18 months of storage under the conditions tested, thus confirming the stability of the formulations.

### Example 5. Effect of Arginine, Histidine and Sucrose Concentrations on Viscosity and Stability of Formulations Containing mAb1

Several formulations were prepared containing 100 mg/mL, 125 mg/mL and 150 mg/mL mAb1 and various quantities of histidine, arginine and sucrose. Viscosity and osmolality were measured for each formulation at 25°C. The results are summarized in Table 13.

**Table 13**

| **Effect of Arginine, Histidine and Sucrose on Viscosity of mAb1 Formulations (All formulations contain 10 mM Acetate, pH 5.0, and 0.2% polysorbate 20)** | | | | | |
|---|---|---|---|---|---|
| **mAb1 (mg/mL)** | **[Histidine] (mM)** | **[Arginine] (mM)** | **[Sucrose] (%)** | **Viscosity (cPoise)** | **Osmolality (mOsm)** |
| 100 | 0 | 0 | 10 | ∼13.5 | 440 |
| 100 | 0 | 25 | 10 | ∼9 | 510 |
| 100 | 25 | 0 | 10 | ∼9 | 510 |
| 100 | 0 | 25 | 5 | ∼7 | 270 |
| 100 | 0 | 50 | 10 | ∼7 | 560 |
| 125 | 0 | 0 | 5 | ∼20 | -250 |
| 125 | 0 | 25 | 5 | ∼11 | 290 |
| 125 | 25 | 0 | 5 | ∼12 | 280 |
| 125 | 0 | 50 | 5 | ∼9 | 340 |
| 125 | 25 | 25 | 5 | ∼9 | 330 |
| 125 | 0 | 0 | 2 | ∼16 | 150 |
| 150 | 0 | 0 | 5 | ∼41 | 300 |
| 150 | 25 | 25 | 5 | ∼19 | 360 |
| 150 | 25 | 25 | 2 | ∼15 | 310 |
| 150 | 0 | 75 | 5 | ∼14 | 450 |
| 150 | 0 | 100 | 5 | ∼11 | 510 |

The results presented in Table 13 indicate that adding histidine or arginine at a concentration of 25 mM to mAb1 (at a concentration of 100 mg/mL), significantly reduced the viscosity of the formulation as compared to an antibody formulation containing no histidine and no arginine. In addition, adding arginine at 25 mM to mAb1 (at a concentration of 100 mg/mL), while reducing the sucrose concentration from 10% to 5%, resulted in a further reduction in viscosity. When the mAb1 antibody concentration was raised to 125 mg/mL, both histidine and arginine at 25 mM resulted in significant reduction in viscosity when used alone or together. Furthermore, reducing the sucrose concentration from 5% to 2% with the added histidine and arginine decreased the viscosity of the formulation to an even greater extent. When the mAb1 concentration was increased to 150 mg/mL, a combination of histidine and arginine, each at 25 mM, resulted in a significant reduction in viscosity. Reducing the sucrose from 5% to 2% resulted in a further decrease in viscosity.

Based at least in part on the foregoing, the following Formulation "B" as set forth in Table 14 was prepared.

**Table 14**

| **Component** | **Formulation B** |
|---|---|
| mAb1 | 20 - 100 mg/mL |
| Acetate | 10 mM |
| Polysorbate 20 | 0.05% |
| Sucrose | 8% |
| Arginine | 25 mM |
| pH | 5.0 |

### Example 6. Stability of Formulation A Containing 100 mg/mL mAb1 When Manufactured in a Vial and Syringes

Formulation A (see Table 9) containing 100 mg/mL mAb1 was prepared in a 2 mL glass vial and in two different syringes: regular and low tungsten. The preparations were stored at 5, 25 and 37°C for various amounts of time. The stability of mAb1 following storage was measured by SE-HPLC and CEX-HPLC. The results are shown in Table 15. (An increase in percent acidic species is consistent with deamidation of the antibody and is thus considered an undesired phenomenon with respect to the pharmaceutical formulations of the present invention). As shown in this table, Formulation A containing 100 mg/mL mAb1, stored at 5°C in a glass vial or syringe, remained relatively stable for at least 18 months, and was slightly less stable at 25° or 37° at the later times tested.

**Table 15**

| **Stability of Formulation A Containing 100 mg/mL mAb1 in Vial and Syringe** | | | | | | | |
|---|---|---|---|---|---|---|---|
| | | **2 mL Glass Vial** | | **Regular Syringe** | | **Low Tungsten Syringe** | |
| **Temp** | **Time** | **% Native (SE-HPLC)** | **% Acidic (CEX-HPLC)** | **% Native (SE-HPLC)** | **% Acidic (CEX-HPLC)** | **% Native (SE-HPLC)** | **% Acidic (CEX-HPLC)** |
| - | Start | 98.2 | 14.4 | 98.2 | 14.1 | 98.2 | 14.3 |
| 37°C | 7 days | 97.6 | 13.9 | 97.6 | 14.0 | 97.8 | 13.9 |
| 37°C | 14 days | 97.2 | 14.7 | 97.3 | 14.6 | 97.3 | 14.7 |
| 37°C | 28 days | 96.7 | 15.7 | 96.6 | 15.5 | 96.5 | 15.5 |
| 25°C | 1 month | 97.7 | 13.1 | 97.5 | 13.5 | 97.9 | 13.3 |
| 25°C | 3 month | 97.3 | 14.9 | 97.2 | 14.9 | 97.1 | 14.8 |
| 25°C | 6 month | 96.9 | 17.4 | 97.1 | 16.9 | 96.7 | 17.4 |
| 5°C | 1 month | 97.9 | 14.8 | 98.1 | 13.7 | 98.0 | 14.0 |
| 5°C | 3 month | 97.8 | 13.7 | 97.6 | 13.9 | 97.7 | 13.5 |
| 5°C | 6 month | 98.2 | 14.0 | 98.1 | 13.5 | 98.0 | 13.7 |
| 5°C | 9 month | 97.4 | 13.4 | 97.6 | 13.1 | 97.6 | 13.2 |
| 5°C | 12 month | 98.1 | 13.8 | 98.1 | 13.5 | 98.2 | 13.1 |
| 5°C | 18 month | 97.6 | 14.0 | 97.6 | 14.1 | 97.7 | 14.0 |

### Example 7. Stability of Formulation B Containing 100 mg/mL mAb1 When Manufactured in a Vial and Syringes

Formulation B (see Table 14) containing 100 mg/mL mAb1 was prepared in a 2 mL glass vial and in two different syringes: regular and low tungsten. The preparations were stored at 5, 25 and 37°C for various amounts of time. The stability of mAb1 following storage was measured by SE-HPLC and CEX-HPLC. The results are shown in Table 16. (As noted previously, an increase in percent acidic species is consistent with deamidation of the antibody and is thus considered an undesired phenomenon with respect to the pharmaceutical formulations of the present invention). As shown in this table, Formulation B containing 100 mg/mL mAb1, stored at 5°C in a glass vial or syringe, remained relatively stable for at least 12 months, and was slightly less stable at 25° or 37° at all time points tested.

**Table 16**

| **Stability of Formulation B Containing 100 mg/mL mAb1 in Vial and Syringe** | | | | | | | |
|---|---|---|---|---|---|---|---|
| | | **2 mL Glass Vial** | | **Regular Syringe** | | **Low Tungsten Syringe** | |
| **Temp** | **Time** | **% Native (SE-HPLC)** | **% Acidic (CEX-HPLC)** | **% Native (SE-HPLC)** | **% Acidic (CEX-HPLC)** | **% Native (SE-HPLC)** | **% Acidic (CEX-HPLC)** |
| - | Start | 98.2 | 14.2 | 98.1 | 14.4 | 98.2 | 14.3 |
| 37°C | 7 days | 97.4 | 14.3 | 97.4 | 14.3 | 97.6 | 14.1 |
| 37°C | 14 days | 97.0 | 14.9 | 96.9 | 14.8 | 96.9 | 14.8 |
| 37°C | 28 days | 96.4 | 16.3 | 96.4 | 16.2 | 96.4 | 15.9 |
| 25°C | 1 month | 97.7 | 13.6 | 97.8 | 13.7 | 97.7 | 13.6 |
| 25°C | 3 month | 96.7 | 14.7 | 96.9 | 14.7 | 96.8 | 14.8 |
| 25°C | 6 month | 96.6 | 15.4 | 96.6 | 15.5 | 96.6 | 15.6 |
| 5°C | 1 month | 98.1 | 13.6 | 98.0 | 13.7 | 98.1 | 13.5 |
| 5°C | 2 month | 97.8 | 14.3 | 97.9 | 14.3 | 97.9 | 14.2 |
| 5°C | 3 month | 97.7 | 14.3 | 97.3 | 14.4 | 97.4 | 14.2 |
| 5°C | 6 month | 97.4 | 14.4 | 97.5 | 14.5 | 97.6 | 14.5 |
| 5°C | 9 month | 97.8 | 14.2 | 97.8 | 14.1 | 97.7 | 14.1 |
| 5°C | 12 month | 98.0 | 15.1 | 98.1 | 14.7 | 98.2 | 14.7 |
| 5°C | 18 month | 97.7 | 14.3 | 97.7 | 39.8 | 97.7 | 14.1 |

### Example 8: Stability of mAb1 Formulations in Prefilled Syringes

A series of experiments was carried out to assess the stability of different mAb1 formulations in prefilled syringes. For these experiments various luer and staked needle, regular-tungsten and low-tungsten syringes were used in combination with different types of plungers (coated and uncoated) and tip-caps. The formulations were tested for stability after storage in prefilled syringes at 37°C, 25°C and 5°C for various amounts of time (ranging from 7 days to 6 months, depending on the conditions tested).

The following formulations of mAb1 were tested for stability in prefilled syringes in this Example: (1) Formulation A (see Table 9) containing 100 mg/mL mAb1 in staked prefilled syringe #1 described in Table 17; (2) Formulation A containing 100 mg/mL mAb1 in staked prefilled syringe #2 described in Table 18; (3) Formulation A containing 20 mg/mL mAb1 in staked prefilled syringe #1 described in Table 19; (4) Formulation A containing 20 mg/mL mAb1 in staked prefilled syringe #2 described in Table 20; (5) Formulation A containing 100 mg/mL of mAb1 in staked prefilled syringe #3 described in Table 21; and (6) Formulation A containing 6 mg/mL of mAb1 in staked prefilled syringe #3 described in Table 22.

Syringe #1 is a BD 1mL long 29 gauge x ½ inch Physiolis low tungsten syringe; syringe #2 is a Schott 1 mL long SN CF 29 gauge x ½ inch syringe; and syringe #3 is a Daikyo Seiko CZ® (Crystal Zenith) 1 mL std 30 gauge x ½ inch syringe.

Stability was assessed by the following parameters: (a) visual analysis; (b) turbidity (OD₄₀₅ₙₘ); (c) percent recovery by RP-HPLC; (d) percent native mAb1 by SE-HPLC; (e) percent main peak mAb1 by CEX-HPLC; and (f) percent acidic species by CEX-HPLC.

The results from a representative experiment assessing the stability of Formulation A, containing 100 mg/mL mAb1 in syringe #1 is shown in Table 17 below.

**Table 17**

| **Stability of Formulation A containing 100 mg/mL mAb1 in Staked Needle Prefilled Syringe #1** | | | | | | | |
|---|---|---|---|---|---|---|---|
| **Syringe #1 Description:** | | | | | | | |
| Syringe: | | BD 1 mL long 29ga x 1/2" Physiolis, Low Tungsten | | | | | |
| Plunger: | | Hypak FluroTec® 4023/50 | | | | | |
| Tip Cap: | | BD 260 | | | | | |
| Siliconization: | | Sprayed | | | | | |

| **Temp** | **Time** | Visual Analysis | Turbidity (OD_{405 nm}) | % Recovery | % Native mAb1 (SE-HPLC) | % Main Peak (CEX-HPLC) | % Acidic Species (CEX-HPLC) |
|---|---|---|---|---|---|---|---|
| -- | Start | Pass | 0.00 | 100 | 98.2 | 40.2 | 14.2 |
| 37°C | 7 days | Pass | 0.00 | 101 | 97.8 | 37.6 | 13.9 |
| 37°C | 14 days | Pass | 0.00 | 100 | 97.3 | 37.0 | 14.7 |
| 37°C | 28 days | Pass | 0.00 | 96 | 96.5 | 34.9 | 15.5 |
| 25°C | 1 month | Pass | 0.00 | 97 | 97.9 | 36.9 | 13.3 |
| 25°C | 2 months | Pass | 0.00 | 103 | 97.0 | 38.6 | 14.9 |
| 25°C | 3 months | Pass | 0.00 | 100 | 97.1 | 35.5 | 14.8 |
| 25°C | 6 months | Pass | 0.00 | 104 | 96.7 | 36.8 | 17.4 |
| 5°C | 1 month | Pass | 0.00 | 96 | 98.0 | 39.0 | 13.8 |
| 5°C | 2 months | Pass | 0.00 | 103 | 97.8 | 39.3 | 13.6 |
| 5°C | 3 months | Pass | 0.00 | 100 | 97.7 | 39.2 | 13.5 |
| 5°C | 6 months | Pass | 0.00 | 100 | 98.0 | 39.2 | 13.7 |
| 5°C | 9 months | Pass | 0.00 | 100 | 97.6 | 39.1 | 13.2 |
| 5°C | 12 months | Pass | 0.00 | 102 | 98.2 | 40.9 | 13.1 |
| 5°C | 18 months | Pass | 0.00 | 100 | 97.7 | 39.6 | 14.0 |

The results from a representative experiment assessing the stability of Formulation A, containing 100 mg/mL mAb1 in syringe #2 is shown in Table 18 below.

**Table 18**

| **Stability of Formulation A containing 100 mg/mL mAb1 in Staked Needle Prefilled Syringe #2** | | | | | | | |
|---|---|---|---|---|---|---|---|
| **Syringe #2 Description:** | | | | | | | |
| Syringe: | | Schott 1 mL Long SN CF 29ga x 1/2" | | | | | |
| Plunger: | | West FluroTec® 4023/50 | | | | | |
| Tip Cap: | | Stelmi 4800 w/RNS | | | | | |
| Siliconization: | | Sprayed | | | | | |

| **Temp** | **Time** | Visual Analysis | Turbidity (OD_{405 nm}) | % Recovery | % Native mAb1 (SE-HPLC) | % Main Peak (CEX-HPLC) | % Acidic Species (CEX-HPLC) |
|---|---|---|---|---|---|---|---|
| -- | Start | Pass | 0.00 | 100 | 98.3 | 40.3 | 14.2 |
| 37°C | 7 days | Pass | 0.00 | 102 | 97.5 | 37.8 | 14.0 |
| 37°C | 14 days | Pass | 0.00 | 102 | 97.3 | 37.4 | 14.7 |
| 37°C | 28 days | Pass | 0.00 | 97 | 96.6 | 34.6 | 15.4 |
| 25°C | 1 month | Pass | 0.00 | 98 | 97.8 | 36.8 | 13.4 |
| 25°C | 2 months | Pass | 0.00 | 103 | 97.1 | 38.8 | 14.9 |
| 25°C | 3 months | Pass | 0.00 | 101 | 97.1 | 35.6 | 14.8 |
| 25°C | 6 months | Pass | 0.00 | 103 | 96.8 | 37.4 | 17.5 |
| 5°C | 1 month | Pass | 0.00 | 97 | 97.8 | 39.8 | 13.8 |
| 5°C | 2 months | Pass | 0.00 | 103 | 97.8 | 39.4 | 13.9 |
| 5°C | 3 months | Pass | 0.00 | 101 | 97.6 | 39.8 | 13.8 |
| 5°C | 6 months | Pass | 0.00 | 101 | 98.1 | 40.0 | 13.9 |
| 5°C | 9 months | Pass | 0.00 | 102 | 97.6 | 39.7 | 13.4 |
| 5°C | 12 months | Pass | 0.00 | 100 | 98.2 | 41.1 | 13.0 |
| 5°C | 18 months | Pass | 0.00 | 102 | 97.7 | 39.4 | 13.9 |

The results from a representative experiment assessing the stability of Formulation A, containing 20 mg/mL mAb1 in syringe #1 is shown in Table 19 below.

**Table 19**

| **Stability of Formulation A containing 20 mg/mL mAb1 in Staked Needle Prefilled Syringe #1** | | | | | | | |
|---|---|---|---|---|---|---|---|
| **Syringe #1 Description:** | | | | | | | |
| Syringe: | | BD 1 mL long 29ga x 1/2" Physiolis, Low Tungsten | | | | | |
| Plunger: | | Hypak FluroTec® 4023/50 | | | | | |
| Tip Cap: | | BD 260 | | | | | |
| Siliconization: | | Sprayed | | | | | |

| **Temp** | **Time** | Visual Analysis | Turbidity (OD_{405 nm}) | % Recovery | % Native mAb1 (SE-HPLC) | % Main Peak (CEX-HPLC) | % Acidic Species (CEX-HPLC) |
|---|---|---|---|---|---|---|---|
| -- | Start | Pass | 0.00 | 100 | 98.9 | 44.5 | 15.9 |
| 37°C | 7 days | Pass | 0.00 | 100 | 98.4 | 42.1 | 15.7 |
| 37°C | 14 days | Pass | 0.00 | 99 | 98.3 | 41.8 | 16.2 |
| 37°C | 28 days | Pass | 0.00 | 98 | 97.6 | 39.2 | 17.5 |
| 25°C | 1 month | Pass | 0.00 | 96 | 98.4 | 41.4 | 15.3 |
| 25°C | 2 months | Pass | 0.00 | 100 | 98.1 | 41.8 | 16.0 |
| 25°C | 3 months | Pass | 0.00 | 99 | 98.0 | 42.7 | 17.0 |
| 25°C | 6 months | Pass | 0.01 | 99 | 98.0 | 42.0 | 19.4 |
| 5°C | 1 month | Pass | 0.00 | 97 | 98.2 | 44.0 | 15.7 |
| 5°C | 2 months | Pass | 0.00 | 99 | 98.5 | 44.5 | 15.8 |
| 5°C | 3 months | Pass | 0.00 | 100 | 98.4 | 44.4 | 15.8 |
| 5°C | 6 months | Pass | 0.00 | 99 | 98.6 | 44.1 | 15.6 |
| 5°C | 9 months | Pass | 0.00 | 101 | 98.2 | 44.0 | 15.1 |
| 5°C | 12 months | Pass | 0.00 | 101 | 98.5 | 45.4 | 15.9 |
| 5°C | 18 months | Pass | 0.00 | 101 | 98.2 | 43.5 | 15.3 |

The results from a representative experiment assessing the stability of Formulation A, containing 20 mg/mL mAb1 in syringe #2 is shown in Table 20 below.

**Table 20**

| **Stability of Formulation A containing 20 mg/mL mAb1 in Staked Needle Prefilled Syringe #2** | | | | | | | |
|---|---|---|---|---|---|---|---|
| **Syringe #2 Description:** | | | | | | | |
| Syringe: | | Schott 1 mL Long SN CF 29ga x 1/2" | | | | | |
| Plunger: | | West FluroTec® 4023/50 | | | | | |
| Tip Cap: | | Stelmi 4800 w/RNS | | | | | |
| Siliconization: | | Sprayed | | | | | |

| **Temp** | **Time** | Visual Analysis | Turbidity (OD_{405 nm}) | % Recovery | % Native mAb1 (SE-HPLC) | % Main Peak (CEX-HPLC) | % Acidic Specie s (CEX-HPLC) |
|---|---|---|---|---|---|---|---|
| -- | Start | Pass | 0.00 | 100 | 98.9 | 44.5 | 15.9 |
| 37°C | 7 days | Pass | 0.00 | 101 | 98.4 | 42.1 | 15.6 |
| 37°C | 14 days | Pass | 0.00 | 100 | 98.2 | 41.8 | 16.2 |
| 37°C | 28 days | Pass | 0.00 | 98 | 97.6 | 39.3 | 17.4 |
| 25°C | 1 month | Pass | 0.00 | 97 | 98.3 | 41.5 | 15.2 |
| 25°C | 2 months | Pass | 0.00 | 99 | 98.2 | 41.9 | 16.0 |
| 25°C | 3 months | Pass | 0.00 | 100 | 98.0 | 42.7 | 16.9 |
| 25°C | 6 months | Pass | 0.00 | 99 | 97.9 | 42.1 | 19.5 |
| 5°C | 1 month | Pass | 0.00 | 96 | 98.4 | 44.7 | 15.6 |
| 5°C | 2 months | Pass | 0.00 | 99 | 98.5 | 44.3 | 15.9 |
| 5°C | 3 months | Pass | 0.00 | 99 | 98.4 | 44.7 | 15.7 |
| 5°C | 6 months | Pass | 0.00 | 100 | 98.7 | 44.2 | 15.5 |
| 5°C | 9 months | Pass | 0.00 | 102 | 98.4 | 43.8 | 15.1 |
| 5°C | 12 months | Pass | 0.00 | 102 | 98.6 | 45.2 | 15.9 |
| 5°C | 18 months | Pass | 0.00 | 102 | 98.3 | 43.2 | 15.2 |

The results from a representative experiment assessing the stability of Formulation A, containing 100 mg/mL mAb1 in syringe #3 is shown in Table 21 below.

**Table 21**

| **Stability of Formulation A containing 100 mg/mL mAb1 in Staked Needle Prefilled Syringe #3** | | | | | | | |
|---|---|---|---|---|---|---|---|
| **Syringe #3 Description:** | | | | | | | |
| Syringe: | | Daikyo Seiko CZ 1 mL std 30ga x 1/2 " | | | | | |
| Plunger: | | Daikyo D-21-6-1 FluroTec® Coated | | | | | |
| Tip Cap: | | 7028 | | | | | |
| Siliconization: | | N/A | | | | | |

| **Temp** | **Time** | Visual Analysis | Turbidity (OD_{405 nm}) | % Recovery | % Native mAb1 (SE-HPLC) | % Main Peak (CEX-HPLC) | % Acidic Species (CEX-HPLC) |
|---|---|---|---|---|---|---|---|
| -- | Start | Pass | 0.00 | 100 | 97.5 | 38.5 | 13.6 |
| 37°C | 7 days | Pass | 0.00 | 101 | 97.5 | 40.0 | 14.0 |
| 37°C | 14 days | Pass | 0.00 | 102 | 97.3 | 39.5 | 15.2 |
| 37°C | 28 days | Pass | 0.00 | 101 | 96.4 | 38.3 | 15.7 |
| 25°C | 1 month | Pass | 0.00 | 105 | 97.8 | 38.3 | 13.6 |
| 25°C | 2 months | Pass | 0.00 | 102 | 97.2 | 37.4 | 14.9 |
| 25°C | 3 months | Pass | 0.00 | 102 | 97.2 | 37.2 | 14.6 |
| 5°C | 1 month | Pass | 0.00 | 104 | 98.2 | 38.1 | 13.3 |
| 5°C | 2 months | Pass | 0.00 | 101 | 97.9 | 38.1 | 13.4 |
| 5°C | 3 months | Pass | 0.00 | 101 | 97.8 | 38.4 | 13.1 |
| 5°C | 6 months | Pass | 0.00 | 106 | 97.8 | 39.6 | 13.4 |
| 5°C | 9 months | Pass | 0.00 | 105 | 97.9 | 40.8 | 13.4 |
| 5°C | 12 months | Pass | 0.00 | 112 | 97.6 | 38.3 | 13.5 |

The results from a representative experiment assessing the stability of Formulation A, containing 6 mg/mL mAb1 in syringe #3 is shown in Table 22 below.

**Table 22**

| **Stability of Formulation A containing 6 mg/mL mAb1 in Staked Needle Prefilled Syringe #3** | | | | | | | |
|---|---|---|---|---|---|---|---|
| **Syringe #3 Description:** | | | | | | | |
| Syringe: | | Daikyo Seiko CZ 1 mL std 30ga x 1/2 " | | | | | |
| Plunger: | | Daikyo D-21-6-1 FluroTec® Coated | | | | | |
| Tip Cap: | | 7028 | | | | | |
| Siliconization: | | N/A | | | | | |

| **Temp** | **Time** | Visual Analysis | Turbidity (OD_{405 nm}) | % Recovery | % Native mAb1 (SE-HPLC) | % Main Peak (CEX-HPLC) | % Acidic Species (CEX-HPLC) |
|---|---|---|---|---|---|---|---|
| -- | Start | Pass | 0.00 | 100 | 98.8 | 45.6 | 16.8 |
| 37°C | 7 days | Pass | 0.00 | 101 | 98.7 | 46.2 | 17.2 |
| 37°C | 14 days | Pass | 0.00 | 101 | 98.5 | 45.8 | 18.2 |
| 37°C | 28 days | Pass | 0.00 | 101 | 98.3 | 44.5 | 19.8 |
| 25°C | 1 month | Pass | 0.00 | 106 | 98.8 | 45.7 | 17.3 |
| 25°C | 2 months | Pass | 0.00 | 103 | 98.8 | 44.3 | 18.1 |
| 25°C | 3 months | Pass | 0.00 | 101 | 98.3 | 42.9 | 18.6 |
| 5°C | 1 month | Pass | 0.00 | 105 | 99.0 | 46.0 | 16.6 |
| 5°C | 2 months | Pass | 0.00 | 102 | 98.8 | 45.6 | 16.8 |
| 5°C | 3 months | Pass | 0.00 | 101 | 98.8 | 45.7 | 16.3 |
| 5°C | 6 months | Pass | 0.00 | 105 | 98.5 | 46.7 | 16.5 |
| 5°C | 9 months | Pass | 0.00 | 106 | 98.8 | 47.8 | 17.2 |
| 5°C | 12 months | Pass | 0.00 | 115 | 97.8 | 47.6 | 17.5 |

The results from this set of experiments demonstrate that the different formulations remain relatively stable in prefilled syringes, especially when stored at temperatures of 25°C and below, for one month or greater.

### Example 9: Stability of Formulations Containing Low Concentrations of mAb1 in Glass Vials

Real-time and accelerated stability of 0.2, 0.5, 1, and 2 mg/mL mAb1 is being assessed in glass vials, the results to date of which are shown in Tables 23 to 27. For these experiments, the stability of mAb1 is being examined in Type 1, borosilicate glass vials manufactured by Schott. The formulation of the mAb1 used in Examples 9 through 12 is similar to that described in Table 9, except that the concentrations of antibody used were lower than previously tested and vary throughout the testing periods. The exact concentrations of mAb1 used in each experiment are noted within each table below. The formulations were tested for stability after storage in glass vials at 45°C, 25°C and 5°C for various amounts of time (ranging from 7 days to 6 months, depending on the conditions tested).

The results to date demonstrate increased degradation (precipitation, aggregation, cleavage, and charge variants) of the 0.2 and 0.5 mg/mL formulations after incubation at 45°C for 7 to 14 days. Furthermore, there was an increase in aggregation for formulations at all concentrations ≤ 2 mg/mL when incubated at 45°C for 28 days (> 15% vs -2% for formulations ≥ 6 mg/mL). Precipitation was observed when the 0.2 mg/mL formulation was stored at 5°C for 6 months. There was no significant degradation/precipitation observed when 0.5, 1, and 2 mg/mL formulations were stored at 5°C for 6 months.

**Table 23**

| **Stability of Low Concentration mAb1 in Glass Vials (45° incubation)** | | | | |
|---|---|---|---|---|
| **Time (days)** | **% mAb1 Recovered (RP-HPLC)** | | | |
| | **Concentration of mAb1 (mg/ml vial)** | | | |
| | **0.2** | **0.5** | **1.0** | **2.0** |
| 0 | 100 | 100 | 100 | 100 |
| 7 | 91 | 97 | 103 | 109 |
| 14 | 67 | 94 | 101 | 108 |
| 28 | 40 | 82 | 100 | 106 |
| **Formulation: 8% Sucrose, 0.05% Polysorbate 20, 10mM acetate, pH5.0** | | | | |

**Table 24**

| **Stability of low Concentration mAb1 in Glass Vials (25° Incubation)** | | | | |
|---|---|---|---|---|
| **Time (days)** | **% Native mAb1 Recovered (SE-HPLC)** | | | |
| | **Concentration of mAb1 (mg/ml vial)** | | | |
| | **0.2** | **0.5** | **1.0** | **2.0** |
| 0 | 98.1 | 98.4 | 98.6 | 98.5 |
| 7 | 98.3 | 98.6 | 98.6 | 98.7 |
| 14 | 98.1 | 98.7 | 98.6 | 98.7 |
| 28 | 97.6 | 98.5 | 98.6 | 98.7 |
| 56 | 92.2 | 96.6 | 98.1 | 98.5 |
| **Formulation: 8% Sucrose, 0.05% Polysorbate 20, 10mM acetate, pH5.0** | | | | |

**Table 25**

| **Stability of low concentration mAb1 in glass vials (5° Incubation)** | | | | |
|---|---|---|---|---|
| **Time (months)** | **% mAb1 Recovered (RP-HPLC)** | | | |
| | **Concentration of mAb1 (mg/ml vial)** | | | |
| | **0.2** | **0.5** | **1.0** | **2.0** |
| 0 | 100 | 100 | 100 | 100 |
| 1 | 101 | 97 | 105 | 109 |
| 2 | 99 | 100 | 107 | 110 |
| 3 | 105 | 99 | 105 | 111 |
| 6 | 80 | 97 | 102 | 106 |
| **Formulation: 8% Sucrose, 0.05% Polysorbate 20, 10mM acetate, pH5.0** | | | | |

**Table 26**

| **Stability of low concentration mAb1 in glass vials (5° Incubation)** | | | | |
|---|---|---|---|---|
| **Time (months)** | **% Native mAb1 Recovered (SE-HPLC)** | | | |
| | **Concentration of mAb1 (mg/ml vial)** | | | |
| | **0.2** | **0.5** | **1.0** | **2.0** |
| 0 | 98.1 | 98.4 | 98.6 | 98.5 |
| 1 | 97.7 | 98.6 | 98.7 | 98.8 |
| 2 | 97.8 | 98.3 | 98.3 | 98.7 |
| 3 | 98.2 | 98.7 | 98.7 | 98.3 |
| 6 | 97.6 | 98.4 | 98.6 | 98.5 |
| **Formulation: 8% Sucrose, 0.05% Polysorbate 20, 10mM acetate, pH5.0** | | | | |

**Table 27: Stability of low concentration mAb1 in PFS syringes or glass vials (45° incubation)**

| **A. Glass Vials** | | | | |
|---|---|---|---|---|
| **Time (days)** | **% Native mAb1 Recovered (SE-HPLC)** | | | |
| | **Concentration of mAb1 (mg/ml vial)** | | | |
| | **0.2** | **0.5** | **1.0** | **2.0** |
| 0 | 98.1 | 98.4 | 98.6 | 98.5 |
| 7 | 90.4 | 97.2 | 98.3 | 98.2 |
| 14 | 76.1 | 92.0 | 96.1 | 97.8 |
| 28 | 42.9 | 51.0 | 83.7 | 81.7 |
| **Formulation: 8% Sucrose, 0.05% Polysorbate 20, 10mM acetate, pH5.0** | | | | |
| **B. Syringe** | | | | |
| **Time (days)** | **% Native mAb1 Recovered (SE-HPLC)** | | | |
| | **Concentration of mAb1 (mg/ml vial)** | | | |
| | **0.2** | **0.5** | **1.0** | **2.0** |
| 0 | 98.1 | 98.6 | 98.5 | 98.4 |
| 7 | 97.6 | 98.1 | 98.2 | 98.1 |
| 14 | 97.9 | 97.9 | 97.8 | 97.8 |
| 28 | 96.9 | 96.8 | 96.7 | 96.6 |
| **Formulation: 8% Sucrose, 0.05% Polysorbate 20, 10mM acetate, pH5.0** | | | | |

### Example 10: Stability of Formulation Containing Low Concentrations of mAb1 in BD and Ompi Syringes

Real-time and accelerated stability of 0.2 and 0.6 mg/mL mAb1 is being assessed in BD and Ompi syringes. Both syringes are 1 mL long in size, manufactured with low tungsten, and have a 27 gauge X ½ inch, thin-walled, staked needle. The BD syringe contains 0.8 mg of silicone, applied by spraying from the top of the flange. The Ompi syringe contains 0.5 mg of silicone and is applied with diving nozzle technology, which gives a more uniform coating along the length of the syringe barrel. The formulations were tested for stability after storage in both prefilled syringes at 45°C, 25°C and 5°C for various amounts of time (ranging from 7 days to 6 months, depending on the conditions tested).

The results, which are shown in Tables 28 to 30, demonstrated that stability of mAb1 at 45°C in the BD syringe is greatly improved compared to a Type 1, borosilicate glass vial as determined by RP-HPLC (precipitation) and SE-HPLC analysis (aggregation and cleavage species).

The stability of mAb1 at 45°C in the Ompi syringe improved compared to Type 1, borosilicate glass vials, but was significantly less stable compared to incubation in the BD syringe. These data suggest that silicone may be blocking the formulation's interaction with the glass surface, thus improving stability. Since the BD syringe contains more silicone along the glass syringe barrel than the Ompi syringe, this may explain why the formulation is more stable in the BD syringe compared to the Ompi syringe.

No significant degradation was observed when mAb1 was stored in the BD and Ompi syringes at 5°C or 25°C for 6 months as determined by SE-HPLC and CEX-HPLC analysis.

**Table 28**

| **Stability of 0.6 mg/mL mAb1 in BD syringes, Ompi Syringe or glass vials (45° Incubation)** | | | |
|---|---|---|---|
| **Time (days)** | **% Native mAb1 Recovered (SE-HPLC)** | | |
| | **Type 1 Glass Vial** | **BD Syringe** | **Ompi Syringe** |
| 0 | 98.6 | 98.6 | 98.9 |
| 7 | 98.2 | 98.4 | 98.5 |
| 14 | 97.7 | 98.0 | 98.5 |
| 28 | 66.1 | 97.1 | 84.5 |
| 54 | - | 95.6 | 24.5 |
| **Formulation: 8% Sucrose, 0.05% Polysorbate 20, 10mM acetate, pH5.0** | | | |

**Table 29**

| **Stability of low concentration mAb1 in BD and Ompi Syringes (5° Incubation; SE-HPLC)** | | | | | | |
|---|---|---|---|---|---|---|
| | **% Native mAb1 Recovered (SE-HPLC)** | | | | | |
| | **Concentration of mAb1 (mg/mL)** | | | | | |
| **Time (months)** | **Vial** | | **BD** | | **Ompi** | |
| | **0.2** | **0.6** | **0.2** | **0.6** | **0.2** | **0.6** |
| 0 | 98.6 | 98.6 | 98.4 | 98.6 | 98.7 | 98.9 |
| 1 | 98.7 | 99.0 | 98.5 | 98.9 | 98.5 | 98.9 |
| 2 | 98.3 | 98.9 | 98.7 | 98.9 | 98.6 | 98.9 |
| 3 | 98.4 | 98.6 | 98.4 | 98.7 | 98.3 | 98.7 |
| 6 | 98.3 | 98.6 | 98.3 | 98.7 | 98.2 | 98.7 |
| **Formulation: 8% Sucrose, 0.05% Polysorbate 20, 10mM acetate, pH5.0** | | | | | | |

**Table 30**

| **Stability of low concentration mAb1 in Vial, BD and CZ Syringes (45° Incubation; RP-HPLC)** | | | | | | |
|---|---|---|---|---|---|---|
| **% mAb1 Recovered (RP-HPLC)** | | | | | | |
| | **Concentration of mAb1 (mg/mL)** | | | | | |
| **Time (days)** | **Vial** | | **BD** | | **CZ** | |
| | **0.2** | **0.6** | **0.2** | **0.6** | **0.2** | **0.6** |
| 0 | 100 | 100 | 100 | 100 | 100 | 100 |
| 7 | 92 | 103 | 96 | 107 | 99 | 106 |
| 14 | 88 | 96 | 96 | 99 | 104 | 100 |
| 28 | 39 | 94 | 85 | 99 | 104 | 101 |
| 42 | - | - | 61 | - | 94 | - |
| 56 | - | - | 55 | - | 96 | - |
| 62 | - | - | - | 88 | - | 98 |
| **Formulation: 8% Sucrose, 0.05% Polysorbate 20, 10mM acetate, pH5.0** | | | | | | |

### Example 11: Stability of Formulation Containing Low Concentrations of mAb1 in Alternative Storage Devices

Studies are ongoing to examine the stability of mAb1 in other storage devices, including: West CZ syringes (cyclic polyolefin), Schott Type 1 plus® glass vials (100 - 200 nm coating of SiO₂ on interior surface of Type 1, borosilicate glass vials), and Schott Type 1, borosilicate glass vials with nitrogen headspace (air removed from vial headspace and replaced with N₂ gas).

The results, as shown in Tables 31 to 36, demonstrate that the stability at 45°C was greatly improved for 0.2 and 0.6 mg/mL formulations in CZ syringes compared to BD syringes as determined by RP-HPLC and SE-HPLC.

Moreover, the stability of the mAb1 formulation at 45°C was greatly improved at 0.2 and 0.6 mg/mL concentrations in Type 1 plus® glass vials compared to Type 1 vials as determined by RP-HPLC and SE-HPLC.

Significant degradation by SE-HPLC was observed for the 0.2 mg/mL formulation in Type 1 glass vials when incubated at 25°C or 5°C for 3 months. No significant degradation was observed for the 0.2 mg/mL formulation in Type 1 plus® glass vials when incubated at 25°C or 5°C for 3 months.

Stability at 45°C was greatly improved for the 0.2 mg/mL formulations in Type 1 glass vials with a nitrogen overlay, compared to Type 1 glass vials with an air headspace as determined by SE-HPLC.

Removal of oxygen from headspace had the greatest stabilizing affect on 0.2 mg/mL mAb1 formulated with 0.05% polysorbate 20 (as in Formulation A) although an increased rate of degradation was still observed compared to a 0.2 mg/mL formulation without polysorbate 20.

**Table 31**

| **Stability of low concentration mAb1 in Vial, BD and CZ Syringes (45° Incubation; SE-HPLC)** | | | | | | |
|---|---|---|---|---|---|---|
| | **% Native mAb1 (SE-HPLC)** | | | | | |
| | **Concentration of mAb1 (mg/mL)** | | | | | |
| **Time (days)** | **Vial** | | **BD** | | **CZ** | |
| | **0.2** | **0.6** | **0.2** | **0.6** | **0.2** | **0.6** |
| 0 | 98.8 | 98.7 | 98.5 | 98.5 | 98.7 | 98.6 |
| 7 | 96.5 | 98.3 | 97.6 | 98.3 | 97.0 | 98.3 |
| 14 | 82.0 | 97.2 | 95.8 | 97.2 | 97.1 | 97.0 |
| 28 | 58.2 | 64.5 | 70.6 | 95.8 | 96.1 | 96.2 |
| 42 | - | - | 48.8 | - | 93.3 | - |
| 56 | - | - | 36.4 | - | 89.6 | - |
| 62 | - | - | - | 93.4 | - | 90.3 |
| **Formulation: 8% Sucrose, 0.05% Polysorbate 20, 10mM acetate, pH5.0** | | | | | | |

**Table 32**

| **Stability of low concentration mAb1 in Type 1 plus® Vial, (45° Incubation; RP-HPLC)** | | | | |
|---|---|---|---|---|
| **% mAb1 Recovered (RP-HPLC)** | | | | |
| **Time (days)** | **Concentration of mAb1 (mg/mL)** | | | |
| | **Type 1 Vial** | | **Type 1 plus® Vial** | |
| | **0.2** | **0.6** | **0.2** | **0.6** |
| 0 | 100 | 100 | 100 | 100 |
| 7 | 92 | 103 | 99 | 106 |
| 14 | 88 | 96 | 100 | 99 |
| 28 | 39 | 94 | 101 | 100 |
| **Formulation: 8% Sucrose, 0.05% Polysorbate 20, 10mM acetate, pH5.0** | | | | |

**Table 33**

| **Stability of low concentration mAb1 in Type 1 plus® Vial (45° Incubation; SE-HPLC)** | | | | |
|---|---|---|---|---|
| **% Native mAb1 (SE-HPLC)** | | | | |
| **Time (days)** | **Concentration of mAb1 (mg/mL)** | | | |
| | **Type 1 Vial** | | **Type 1 plus® Vial** | |
| | **0.2** | **0.6** | **0.2** | **0.6** |
| 0 | 98.8 | 98.7 | 98.7 | 98.6 |
| 7 | 96.5 | 98.3 | 97.3 | 98.3 |
| 14 | 82.0 | 97.2 | 97.4 | 97.2 |
| 28 | 58.2 | 64.5 | 70.6 | 95.2 |
| **Formulation: 8% Sucrose, 0.05% Polysorbate 20, 10mM acetate, pH5.0** | | | | |

**Table 34**

| **Stability of low concentration mAb1 in Type 1 plus® Vial (25° Incubation; SE-HPLC)** | | | | |
|---|---|---|---|---|
| **% Native mAb1 (SE-HPLC)** | | | | |
| **Time (monts)** | **Concentration of mAb1 (mg/mL)** | | | |
| | **Type 1 Vial** | | **Type 1 plus® Vial** | |
| | **0.2** | **0.6** | **0.2** | **0.6** |
| 0 | 98.8 | 98.7 | 98.7 | 98.6 |
| 1 | 98.7 | 98.7 | 98.6 | 98.8 |
| 2 | 98.2 | 98.6 | 98.2 | 98.6 |
| 3 | 92.5 | 97.2 | 97.5 | 97.4 |
| **Formulation: 8% Sucrose, 0.05% Polysorbate 20, 10mM acetate, pH5.0** | | | | |

**Table 35**

| **Stability of low concentration mAb1 in Type 1 Plus® Vial (5° Incubation; SE-HPLC)** | | | | |
|---|---|---|---|---|
| **% Native mAb1 (SE-HPLC)** | | | | |
| **Time (months)** | **Concentration of mAb1 (mg/mL)** | | | |
| | **Type 1 Vial** | | **Type 1 Plus® Vial** | |
| | **0.2** | **0.6** | **0.2** | **0.6** |
| 0 | 98.8 | 98.7 | 98.7 | 98.7 |
| 1 | 98.9 | 98.9 | 98.5 | 98.7 |
| 2 | 98.7 | 98.9 | 98.6 | 98.6 |
| 3 | 96.5 | 97.7 | 97.9 | 97.7 |
| **Formulation: 8% Sucrose, 0.05% Polysorbate 20, 10mM acetate, pH5.0** | | | | |

**Table 36**

| **Stability of 0.2 mg/mL mAb1 in Type 1 Vial with Nitrogen Overlay (45° Incubation; SE-HPLC)** | | |
|---|---|---|
| **% Native mAb1 (SE-HPLC)** | | |
| **Time (months)** | **Air Headspace** | **Nitrogen Headspace** |
| 0 | 97.5 | 97.1 |
| 7 | 95.2 | 95.7 |
| 14 | 84.6 | 96.6 |
| 21 | 60.9 | 96.7 |
| 28 | 53.9 | 96.7 |
| 56 | - | 92.4 |
| **Formulation: 8% Sucrose, 0.05% Polysorbate 20, 10mM acetate, pH5.0** | | |

### Example 12: Effect of Tungsten on Stability of Low Concentration mAb1

Studies are ongoing to examine the stability of mAb1 when spiked with an unfiltered tungsten pin extract. Tungsten pins are used in the manufacturing process of pre-filled syringes to form the hole in the syringe luer. An extract of a tungsten pin used on a manufacturing line by BD was prepared using mAb1 placebo (similar to Formulation A without mAb1). The unfiltered pin extract contains all species of tungsten that may be left behind in the syringe as contaminants (soluble tungsten salts and insoluble tungsten oxides).

The SE-HPLC results, as shown in Tables 37 to 38, demonstrate that the stability at 25°C was reduced compared to a control vial without tungsten for 0.6 mg/mL formulations containing tungsten levels ≥ 500 ppb. Low tungsten syringes typically contain < 500 ppb tungsten whereas regular syringes may contain as much as 2500 ppb tungsten.

No degradation was observed for 0.6 mg/mL formulations with tungsten up to 2500 ppb when incubated at 5°C for 6 months.

**Table 37**

| **Stability of 0.6 mg/mL mAb1 in when stored at 25° in Unfiltered Tungsten Pin Extracts (SE-HPLC)** | | | | | | |
|---|---|---|---|---|---|---|
| | **% Native mAb1 (SE-HPLC)** | | | | | |
| Time (weeks) | **Contro I Vial** | **50 ppb W** | **100 ppb W** | **500 ppb W** | **1000 ppb W** | **2500 ppb W** |
| 0 | 98.7 | 98.8 | 98.9 | 98.8 | 98.8 | 98.7 |
| 1 | 98.7 | 98.6 | 98.7 | 98.6 | 98.7 | 98.7 |
| 2 | 98.6 | 98.6 | 98.6 | 98.4 | 98.4 | 98.4 |
| 4 | 98.5 | 98.5 | 98.5 | 98.6 | 98.2 | 98.5 |
| 8 | 98.6 | 98.7 | 98.6 | 98.2 | 98.3 | 98.6 |
| 26 | 97.4 | 97.8 | 97.5 | 78.6 | 76.5 | 74.8 |
| **Formulation: 0.6 mg/mL mAb1 in 8% Sucrose, 0.05% Polysorbate 20, 10mM acetate, pH5.0** | | | | | | |

**Table 38**

| **Stability of 0.6 mg/mL mAblin when stored at 5° in Unfiltered Tungsten Pin Extracts (SE-HPLC)** | | | | | | |
|---|---|---|---|---|---|---|
| | **% Native mAb1 (SE-HPLC)** | | | | | |
| **Time (weeks)** | **Control Vial** | **50 ppb W** | **100 ppb W** | **500 ppb W** | **1000 ppb W** | **2500 ppb W** |
| 0 | 98.7 | 98.8 | 98.9 | 98.8 | 98.8 | 98.7 |
| 1 | 98.5 | 98.5 | 98.6 | 98.7 | 98.6 | 98.4 |
| 3 | 97.7 | 98.0 | 98.2 | 98.2 | 98.2 | 98.2 |
| 6 | 98.8 | 98.6 | 98.7 | 98.7 | 98.8 | 98.4 |
| **Formulation: 0.6 mg/mL mAb1 in 8% Sucrose, 0.05% Polysorbate 20, 10mM acetate, pH5.0** | | | | | | |

The present invention is not to be limited in scope by the specific embodiments describe herein. Indeed, various modifications of the invention in addition to those described herein will become apparent to those skilled in the art from the foregoing description and the accompanying figures. Such modifications are intended to fall within the scope of the appended claims.

### ASPECTS OF THE INVENTION

1. A pharmaceutical formulation comprising: (i) a human antibody that specifically binds to human nerve growth factor (hNGF); (ii) a non-ionic surfactant; and (iii) a carbohydrate.
2. The pharmaceutical formulation of aspect 1, wherein the carbohydrate is a sugar.
3. The pharmaceutical formulation of aspect 2, wherein the sugar is selected from the group consisting of sucrose, glucose, mannitol, sorbitol, lactose and trehalose.
4. The pharmaceutical formulation of aspect 3, wherein the sugar is sucrose.
5. The pharmaceutical formulation of aspect 1, wherein the non-ionic surfactant is selected from the group consisting of polysorbate 20, polysorbate 80 and polyoxyethylene sorbitan monooleate.
6. The pharmaceutical formulation of aspect 5, wherein the non-ionic surfactant is polysorbate 20.
7. The pharmaceutical formulation of aspect 6, further comprising at least one amino acid selected from the group consisting of histidine or arginine.
8. The pharmaceutical formulation of any of aspects 1-7, further comprising about ImM to about 50 mM acetate.
9. A pharmaceutical formulation comprising: (i) about 0.1 to 100 mg/mL of a human antibody that specifically binds to hNGF; (ii) about 0.01 to 1.0 % of polysorbate 20; and (iii) about 1 to 20% sucrose.
10. The pharmaceutical formulation of aspect 9 comprising: (i) about 0.2 to 75 mg/mL of a human antibody that specifically binds to hNGF; (ii) about 0.02 to 0.5% polysorbate 20; and (iii) about 5 to 10% sucrose.
11. The pharmaceutical formulation of aspect 10, comprising: (i) about 0.6-60 mg/mL of a human antibody that specifically binds to hNGF; (ii) about 0.05% polysorbate 20; and (iii) about 8% sucrose.
12. The pharmaceutical formulation of any of aspects 8-10, further comprising about 1.0 mM to about 50 mM acetate.
13. The pharmaceutical formulation of aspect 12, contained in a glass vial or syringe, or a plastic vial or syringe.
14. The formulation of aspect 13, wherein the glass vial is a silicon dioxide coated glass vial.
15. The formulation of aspect 14, wherein the headspace in the glass vial is filled with an inert gas to remove oxygen.
16. The formulation of aspect 15, wherein the inert gas is argon or nitrogen.
17. The pharmaceutical formulation of either one of aspect 8 or 12, contained in an autoinjector or microinfusor.
18. The pharmaceutical formulation of aspect 13, wherein the syringe comprises a fluorocarbon-coated plunger.
19. The pharmaceutical formulation of aspect 18, wherein the syringe is a low tungsten syringe.
20. The pharmaceutical formulation of aspect 19, wherein the syringe comprises a fluorocarbon-coated plunger.
21. The pharmaceutical formulation of any of aspects 1-20, wherein the human antibody that specifically binds to hNGF comprises a heavy chain variable region (HCVR) and a light chain variable region (LCVR), wherein the HCVR comprises heavy chain complementarity determining regions comprising the amino acid sequences of SEQ ID NOs:6, 8, and 10, and wherein the LCVR comprises light chain complementarity determining regions comprising the amino acid sequences of SEQ ID NOs:14, 16 and 18.
22. The pharmaceutical formulation of aspect 21, wherein the human antibody that specifically binds to hNGF comprises heavy chain and light chain variable region (HCVR /LCVR) amino acid sequence pairs selected from the group consisting of: (i) SEQ ID NQs:20/22; and (ii)SEQ ID NOs:4/12.
23. A pharmaceutical formulation comprising: (i) about 0.1-100 mg/mL of a human antibody that specifically binds to hNGF, wherein said antibody comprises a heavy chain and light chain variable region (HCVR / LCVR) amino acid sequence pair of SEQ ID NQs:20/22; (ii) about 0.05% polysorbate 20; (iii) about 8% sucrose; and (iv) about 10 mM acetate.
24. The pharmaceutical formulation of any of aspects 1 - 23, wherein the pH of the formulation ranges from about 4.5 to 5.6.
25. The pharmaceutical formulation of aspect 24, wherein the pH of the formulation is 5.0.
26. The pharmaceutical formulation of any of aspects 1-25, wherein the formulation is administered intravenously or subcutaneously.
27. The use of the pharmaceutical formulation of any of aspects 1-26 in the preparation of a medicament for the treatment, prevention and/or amelioration of any disease or disorder associated with NGF activity or NGF activation.

## Claims

1. A pharmaceutical formulation contained in a glass vial or syringe, or a plastic vial or syringe, the pharmaceutical formulation, comprising:
(i) 0.2 to 75 mg/mL of a human antibody that specifically binds to human nerve growth factor (hNGF);
(ii) 0.05% polysorbate 20;
(iii) 8% sucrose; and
(iv) 10 mM acetate,
wherein:
- the human antibody that specifically binds to hNGF consists of two heavy and two light chains, each heavy chain comprising a heavy chain variable region (HCVR) comprising the amino acid sequence of SEQ ID NO: 20 and a heavy chain constant region comprising the amino acid sequence of SEQ ID NO: 31, and each light chain comprising a light chain variable region (LCVR) comprising the amino acid sequence of SEQ ID NO: 22 and a light chain constant region;
- the stored formulation, after six months at 5°C, maintains stability such that 97% or more of the antibody is maintained in its native form as detected by sizeexclusion high-performance liquid chromatography; and
- the pH of the formulation is 5.0.

2. The pharmaceutical formulation of claim 1, wherein the formulation comprises 0.6 to 60 mg/mL of the antibody.

3. The pharmaceutical formulation of claim 1 or 2, wherein the formulation is administered intravenously or subcutaneously.

4. The formulation of any one of claims 1-3 , wherein the glass vial is a silicon dioxide coated glass vial.

5. The formulation of claim 4, wherein the headspace in the glass vial is filled with an inert gas to remove oxygen.

6. The formulation of claim 5, wherein the inert gas is argon or nitrogen.

7. The pharmaceutical formulation of any one of claims 1-3, wherein the syringe comprises a fluorocarbon-coated plunger.

8. The pharmaceutical formulation of claim 7, wherein the syringe is a low tungsten syringe.

9. The pharmaceutical formulation of any of claims 1-8 wherein the human antibody that specifically binds to hNGF is present in a concentration of 2 mg/ml.

10. A pharmaceutical formulation contained in an autoinjector or micro-infuser, the pharmaceutical formulation, comprising:
(i) 0.2 to 75 mg/mL of a human antibody that specifically binds to human nerve growth factor (hNGF);
(ii) 0.05% polysorbate 20;
(iii) 8% sucrose; and
(iv) 10 mM acetate,
wherein:
- the human antibody that specifically binds to hNGF consists of two heavy and two light chains, each heavy chain comprising a heavy chain variable region (HCVR) comprising the amino acid sequence of SEQ ID NO: 20 and a heavy chain constant region comprising the amino acid sequence of SEQ ID NO: 31, and each light chain comprising a light chain variable region (LCVR) comprising the amino acid sequence of SEQ ID NO: 22 and a light chain constant region;
- the stored formulation, after six months at 5°C, maintains stability such that 97% or more of the antibody is maintained in its native form as detected by sizeexclusion high-performance liquid chromatography; and
- the pH of the formulation is 5.0.

11. The pharmaceutical formulation of claim 10 wherein the human antibody that specifically binds to hNGF is present in a concentration of 2 mg/ml.

12. Use of the pharmaceutical formulation of any of the preceding claims in the preparation of a medicament for the treatment, prevention and/or amelioration of pain associated with NGF activity or NGF activation.

13. The use according to claim 12, wherein the pain is neurogenic, neuropathic, nociceptic, or is associated with inflammation.

14. The pharmaceutical formulation of any one of claims 1-11 for use in a method of treating, preventing and/or ameliorating pain associated with NGF activity or NGF activation.

15. The formulation for use according to claim 14, wherein the pain is neurogenic, neuropathic, nociceptic or is associated with inflammation.


SEQUENCE LISTING

<110> Scott Walsh
      Terra Potocky
      Daniel Dix
      Renuka Sivendran

<120> Stabilized Formulations Containing Anti-NGF Antibodies

<130> To be assigned

<140> To be assigned
<141> Filed herewith

<150> 61/364,112
<151> 2010-07-14

<160> 31

<170> FastSEQ for Windows Version 4.0

<210> 1
<211> 847
<212> DNA
<213> Homo sapiens

<400> 1
agcgtccgga cccaataaca gttttaccaa gggagcagct ttctatcctg gccacactga 60
ggtgcatagc gtaatgtcca tgttgttcta cactctgatc acagcttttc tgatcggcat 120
acaggcggaa ccacactcag agagcaatgt ccctgcagga cacaccatcc cccaagccca 180
ctggactaaa cttcagcatt cccttgacac tgcccttcgc agagcccgca gcgccccggc 240
agcggcgata gctgcacgcg tggcggggca gacccgcaac attactgtgg accccaggct 300
gtttaaaaag cggcgactcc gttcaccccg tgtgctgttt agcacccagc ctccccgtga 360
agctgcagac actcaggatc tggacttcga ggtcggtggt gctgccccct tcaacaggac 420
tcacaggagc aagcggtcat catcccatcc catcttccac aggggcgaat tctcggtgtg 480
tgacagtgtc agcgtgtggg ttggggataa gaccaccgcc acagacatca agggcaagga 540
ggtgatggtg ttgggagagg tgagcattaa caacagtgta ttcaaacagt acttttttga 600
gaccaagtgc cgggacccaa atcccgttga cagcgggtgc cggggcattg actcaaagca 660
ctggaactca tattgtacca cgactcacac ctttgtcaag gcgctgacca tggatggcaa 720
gcaggctgcc tggcggttta tccggataga tacggcctgt atgtgtgtgc tcagcaggaa 780
ggctgtgaga agagcctgac ctgccgacac gctccctccc cctgcccctt ctacactctc 840
ctgggcc 847

<210> 2
<211> 115
<212> PRT
<213> Homo sapiens

<400> 2
Ser Ser Ser His Pro Ile Phe His Arg Gly Glu Phe Ser Val Val Ser
 1               5                  10                  15
Val Trp Val Gly Asp Lys Thr Thr Ala Thr Asp Ile Lys Gly Lys Glu
            20                  25                  30
Val Met Val Leu Gly Glu Val Asn Ile Asn Asn Ser Val Phe Lys Gln
        35                  40                  45
Tyr Phe Phe Glu Thr Lys Cys Arg Asp Pro Asn Pro Val Asp Ser Gly
    50                  55                  60
Cys Arg Gly Ile Asp Ser Lys His Trp Asn Ser Tyr Cys Thr Thr Thr
65                  70                  75                  80
His Thr Phe Ala Leu Thr Met Asp Gly Lys Gln Ala Ala Trp Arg Phe
                85                  90                  95
Ile Arg Ile Asp Thr Ala Cys Val Cys Val Leu Ser Arg Lys Ala Val

            100                 105                 110
Arg Arg Ala
        115

<210> 3
<211> 357
<212> DNA
<213> Artificial Sequence

<220>
<223> Synthetic

<400> 3
caggtgcagc tggtgcagtc cggcgccgag gtgaagaagc ccggcgcctc cgtgaaggtg 60
tcctgcaagg tgtccggctt caccctgacc gagctgtcca tgcactgggt gcggcaggcc 120
cccggcaagg gcctggagtg gatgggcggc ttcgaccccg aggacggcga gaccatctac 180
gcccagaagt tccagggccg ggtgaccatg accgaggaca cctccaccga caccgcctac 240
atggagctgt cctccctgcg gtccgaggac accgccgtgt actactgctc caccatcttc 300
ggcgtggtga ccaacttcga caactggggc cagggcaccc tggtgaccgt gtcctcc 357

<210> 4
<211> 119
<212> PRT
<213> Artificial Sequence

<220>
<223> Synthetic

<400> 4
Gln Val Gln Leu Val Gln Ser Gly Ala Glu Val Lys Lys Pro Gly Ala
1               5                   10                  15
Ser Val Lys Val Ser Cys Lys Val Ser Gly Phe Thr Leu Thr Glu Leu
            20                  25                  30
Ser Met His Trp Val Arg Gln Ala Pro Gly Lys Gly Leu Glu Trp Met
        35                  40                  45
Gly Gly Phe Asp Pro Glu Asp Gly Glu Thr Ile Tyr Ala Gln Lys Phe
    50                  55                  60
Gln Gly Arg Val Thr Met Thr Glu Asp Thr Ser Thr Asp Thr Ala Tyr
65                  70                  75                  80
Met Glu Leu Ser Ser Leu Arg Ser Glu Asp Thr Ala Val Tyr Tyr Cys
                85                  90                  95
Ser Thr Ile Phe Gly Val Val Thr Asn Phe Asp Asn Trp Gly Gln Gly
            100                 105                 110
Thr Leu Val Thr Val Ser Ser
        115

<210> 5
<211> 24
<212> DNA
<213> Artificial Sequence

<220>
<223> Synthetic

<400> 5
ggattcaccc tcactgaatt atcc 24

<210> 6
<211> 8
<212> PRT
<213> Artificial Sequence

<220>
<223> Synthetic

<400> 6
Gly Phe Thr Leu Thr Glu Leu Ser
1               5


<210> 7
<211> 24
<212> DNA
<213> Artificial Sequence

<220>
<223> Synthetic

<400> 7
tttgatcctg aagatggtga aaca                                              24

<210> 8
<211> 8
<212> PRT
<213> Artificial Sequence

<220>
<223> Synthetic

<400> 8
Phe Asp Pro Glu Asp Gly Glu Thr
1               5


<210> 9
<211> 36
<212> DNA
<213> Artificial Sequence

<220>
<223> Synthetic

<400> 9
tcaacgattt ttggagtggt taccaacttt gacaac                                 36

<210> 10
<211> 12
<212> PRT
<213> Artificial Sequence

<220>
<223> Synthetic

<400> 10
Ser Thr Ile Phe Gly Val Val Thr Asn Phe Asp Asn
1               5                   10


<210> 11
<211> 324
<212> DNA
<213> Artificial Sequence

<220>

<223> Synthetic

<400> 11
gacatccaga tgacccagtc cccctcctcc ctgtccgcct ccgtgggcga ccgggtgacc 60
atcacctgcc gggcctccca ggccatccgg aacgacctgg gctggtacca gcagaagccc 120
ggcaaggccc ccaagcggct gatctacgcc gccttcaacc tgcagtccgg cgtgccctcc 180
cggttctccg gctccggctc cggcaccgag ttcaccctga ccatctcctc cctgcagccc 240
gaggacttcg ccacctacta ctgccagcag tacaaccggt acccctggac cttcggccag 300
ggcaccaagg tggagatcaa gcgg 324

<210> 12
<211> 108
<212> PRT
<213> Artificial Sequence

<220>
<223> Synthetic

<400> 12
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ala Ile Arg Asn Asp
            20                  25                  30
Leu Gly Trp Tyr Gln Gln Lys Pro Gly Lys Ala Pro Lys Arg Leu Ile
        35                  40                  45
Tyr Ala Ala Phe Asn Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Gln Gln Tyr Asn Arg Tyr Pro Trp
                85                  90                  95
Thr Phe Gly Gln Gly Thr Lys Val Glu Ile Lys Arg
            100                 105

<210> 13
<211> 18
<212> DNA
<213> Artificial Sequence

<220>
<223> Synthetic

<400> 13
caggccatta gaaatgat 18

<210> 14
<211> 6
<212> PRT
<213> Artificial Sequence

<220>
<223> Synthetic

<400> 14
Gln Ala Ile Arg Asn Asp
1               5

<210> 15
<211> 9
<212> DNA
<213> Artificial Sequence

<220>
<223> Synthetic

<400> 15
gctgcattc                                                              9

<210> 16
<211> 3
<212> PRT
<213> Artificial Sequence

<220>
<223> Synthetic

<400> 16
Ala Ala Phe
1

<210> 17
<211> 27
<212> DNA
<213> Artificial Sequence

<220>
<223> Synthetic

<400> 17
caacagtata atagataccc gtggacg                                         27

<210> 18
<211> 9
<212> PRT
<213> Artificial Sequence

<220>
<223> Synthetic

<400> 18
Gln Gln Tyr Asn Arg Tyr Pro Trp Thr
1               5

<210> 19
<211> 357
<212> DNA
<213> Artificial Sequence

<220>
<223> Synthetic

<400> 19
caggtgcagc tggtgcagtc tggggctgag gtgaagaagc ctggggcctc agtgaaggtc 60
tcctgcaagg tttccggatt caccctcact gaattatcca ttcactgggt gcgacaggct 120
cctggaaaag ggcttgagtg gatgggaggt tttgatcctg aagatggtga aacaatctac 180
gcacagaagt tccagggcag agtcaccatg accgaggaca catctacaga cacagcctac 240
atggagctga ccagcctgag atcggaagac acggccgtgt attactgttc aacgattttt 300
ggagtggtta ccaactttga caactggggc cagggaaccc tggtcaccgt ctcctca    357

<210> 20
<211> 119
<212> PRT

<213> Artificial Sequence

<220>
<223> Synthetic

<400> 20

<210> 21
<211> 324
<212> DNA
<213> Artificial Sequence

<220>
<223> Synthetic

<400> 21
gacatccaga tgacccagtc tccatcctcc ctgtctgcat ctgcaggaga cagagtcacc 60
atcacttgcc gggcaagtca ggccattaga aatgatttag gctggtatca gcagaaacca 120
gggaaagccc ctaagcgcct gatctatgct gcattcaatt tgcaaagtgg ggtcccatca 180
agattcagcg gcagtggatc tgggacagaa ttcactctca caatcagtag cctgcagcct 240
gaagatcttg caagttatta ctgtcaacag tataatagat acccgtggac gttcggccaa 300
gggaccaagg tggaaatcaa acga                                        324

<210> 22
<211> 108
<212> PRT
<213> Artificial Sequence

<220>
<223> Synthetic

<400> 22
Asp Ile Gln Met Thr Gln Ser Pro Ser Ser Leu Ser Ala Ser Ala Gly
1               5                   10                  15
Asp Arg Val Thr Ile Thr Cys Arg Ala Ser Gln Ala Ile Arg Asn Asp
            20                  25                  30
Leu Gly Trp Tyr Gln Gln Lys Pro Gly Lys Ala Pro Lys Arg Leu Ile
        35                  40                  45
Tyr Ala Ala Phe Asn Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Leu Ala Ser Tyr Tyr Cys Gln Gln Tyr Asn Arg Tyr Pro Trp
                85                  90                  95
Thr Phe Gly Gln Gly Thr Lys Val Glu Ile Lys Arg
            100                 105

<210> 23
<211> 8
<212> PRT
<213> Artificial Sequence

<220>
<223> Synthetic

<220>
<221> VARIANT
<222> (1)...(8)
<223> Xaa = Any amino acid

<400> 23
Xaa Xaa Xaa Xaa Xaa Xaa Xaa Xaa
1               5

<210> 24
<211> 8
<212> PRT
<213> Artificial Sequence

<220>
<223> Synthetic

<220>
<221> VARIANT
<222> (1)...(8)
<223> Xaa = Any amino acid

<400> 24
Xaa Xaa Xaa Xaa Xaa Xaa Xaa Xaa
1               5

<210> 25
<211> 18
<212> PRT
<213> Artificial Sequence

<220>
<223> Synthetic

<220>
<221> VARIANT
<222> (1)...(18)
<223> Xaa = Any amino acid

<400> 25
Xaa Xaa Xaa Xaa Xaa Xaa Xaa Xaa Xaa Xaa Xaa Xaa Xaa Xaa Xaa Xaa
1               5                   10                  15
Xaa Xaa

<210> 26
<211> 6
<212> PRT
<213> Artificial Sequence

<220>
<223> Synthetic

<220>
<221> VARIANT
<222> (1)...(6)
<223> Xaa = Any amino acid

<400> 26
Xaa Xaa Xaa Xaa Xaa Xaa
1               5


<210> 27
<211> 3
<212> PRT
<213> Artificial Sequence

<220>
<223> Synthetic

<220>
<221> VARIANT
<222> (1)...(3)
<223> Xaa = Any amino acid

<400> 27
Xaa Xaa Xaa
1


<210> 28
<211> 9
<212> PRT
<213> Artificial Sequence

<220>
<223> Synthetic

<220>
<221> VARIANT
<222> (1)...(9)
<223> Xaa = Any amino acid

<400> 28
Xaa Xaa Xaa Xaa Xaa Xaa Xaa Xaa Xaa
1               5


<210> 29
<211> 330
<212> PRT
<213> Artificial Sequence

<220>
<223> Synthetic

<400> 29

<210> 30
<211> 327
<212> PRT
<213> Artificial Sequence

<220>
<223> Synthetic

<400> 30
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Ser Cys Pro Ala Pro
            100                 105                 110
Glu Phe Leu Gly Gly Pro Ser Val Phe Leu Phe Pro Pro Lys Pro Lys

115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325

<210> 31
<211> 327
<212> PRT
<213> Artificial Sequence

<220>
<223> Synthetic

<400> 31
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu

195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325